# EUROPÄISCHE PATENTSCHRIFT

(11) **EP 0 308 438 B1**
(45) Veröffentlichungstag und Bekanntmachung des Hinweises auf die Patenterteilung: **29.09.1993**
(21) Anmeldenummer: 88902100.2
(22) Anmeldetag: 10.03.1988
(51) Int. Cl.: C07C 25/24, C07C 15/54, C07C 69/75, C07C 43/215, C07C 255/50, C07C 43/225, C07C 43/30, C07C 43/313, C07D 319/06, C07C 255/36

(54) **Flüssigkristalline Phase, Tolane enthaltend**
Liquid crystal phase containing tolanes
Phase liquide cristalline contenant des tolanes

(30) Priorität: 03.04.1987 DE 3711306; 09.09.1987 WO PCT/EP87/00515
(43) Veröffentlichungstag der Anmeldung: 29.03.1989
(73) Patentinhaber: MERCK PATENT GmbH, D-64271 Darmstadt (DE)
(72) Erfinder: REIFFENRATH, Volker, D-6101 Rossdorf (DE); KRAUSE, Joachim, D-6110 Dieburg (DE); WEBER, Georg, D-6106 Erzhausen (DE); HITTICH, Reinhard, D-6101 Modautal 1 (DE)
(86) Internationale Anmeldenummer: DE8800133
(87) Internationale Veröffentlichungsnummer: WO8807514

(56) Entgegenhaltungen:
- EP-A- 255 700
- EP-A- 276 067
- WO-A-88/02130
- DE-A- 2 504 641
- FR-A- 2 234 261
- GB-A- 2 155 465
- MOLECULAR CHRYSTALS AND LIQUID CHRYSTALS, vol. 62, 1980, New York, NY (US); NGUYEN HUU TINH et al.: "Synthesis and mesorphic properties of the homologous series of 4-alkyl or alkoxy4'-bromo or cyanotolanes", p. 125-140, see p. 127-128
- MOLECULAR CHRYSTALS AND LIQUID CHRYSTALS, vol. 49, 1979, New York, NY (US); NGUYEN HUU TINH et al.: "New series exhibiting pure enantiotropic nematic reentrant compounds at atmospheric pressure", p. 287-291, see the whole article
- CHEMICAL ABSTRACTS, vol. 87, no. 21, 21 November 1977, Columbus, OH (US); J.M. GASCOYNE et al.; "Fluorine-19 nuclear magnetic studies of aromatic compounds. Part 5. Transmission of substituent effects across two aromatic rings connected by C-C and -C- linkages", see p. 492, abstract 166967s
- MOLECULAR CHRYSTALS AND LIQUID CHRYSTALS, vol. 141, 1986, New York, NY (US); H. TAKATSU et al.: "Physical properties of nematic tolans", p. 279-287, see the whole article
- MOLECULAR CHRYSTALS AND LIQUID CHRISTALS, vol. 37, 1976, New York, NY (US); R.J. COX et al.: "The preparation of 4-cyano-4'-alkyltolans. A new series of liquid crystals", pages 241-248, see the whole article
- CHEMICAL ABSTRACTS, vol. 106, no. 22, 01 June 1987, Columbus, Ohio, US; see p. 692, abstract 186666g, & JP-A-61197533
- CHEMICAL ABSTRACTS, vol. 104, no. 12, 24 March 1986, Columbus, Ohio, US, see p. 768, abstract 100055f, & JP-A-60155142
- CHEMICAL ABSTRACTS, vol. 107, no. 22, 30 November 1987, Columbus, Ohio, US, see p. 729, abstract 208997r, & JP-A-61260031

## Beschreibung

Die Erfindung betrifft eine flüssigkristalline Phase enthaltend Tolane der Formel I

R¹-(A¹-Z¹)ₘ-A³-C≡C-A⁴-R² I

worin
- R¹ und R²: jeweils unabhängig voneinander Alkyl mit bis zu 15 C-Atomen, worin auch eine oder zwei nicht benachbarte CH₂-Gruppen durch -O-ersetzt sein können, einer der Reste R¹ und R² auch -OCF₃, CF₃, Fluor oder Chlor,
- A¹: unsubstituiertes 1,4-Phenylen oder trans-1,4-Cyclohexylen,
- Z¹: -CH₂CH₂- oder eine Einfachbindung,
- m: 0 oder 1
und
- A³ und A⁴: jeweils unabhängig voneinander unsubstituiertes oder ein- oder mehrfach durch Fluor substituiertes 1,4-Phenylen
bedeutet, mit den Maßgahen, daß
entweder
a) die Tolane der Formel I eine 2,3-Difluor-1,4-phenylengruppe enthalten, oder
b) R¹ oder R² -CF₃ oder OCF₃ ist,
oder daß
c) im Falle m = 0 und A³ und A⁴ = unsubstituiertes 1,4-Phenylen R¹ Ethoxy, Propoxy, Butoxy, Pentoxy, Hexoxy oder Heptoxy und R² Fluor bedeutet oder einer der Reste R¹ und R² eine Alkylgruppe ist, worin mindestens zwei CH₂-Gruppen durch -O- ersetzt sind,
d) Verbindungen mit m = 0 und A³ und/oder A⁴ = monosubstituiertes 1,4-Phenylen ausgeschlossen sind,
e) im Falle A³ und A⁴ = unsubstituiertes 1,4-Phenylen, m = 1 und Z¹ = eine Einfachbindung, einer der Reste R¹ und R² eine Alkoxygruppe bedeutet und der andere dann Alkyl ist,
und als wichtigste weitere Bestandteile Verbindungen der Formel V enthält,

R⁶-L-G-E-R⁷ V

worin L und E je ein carbo- oder heterocyclisches Ringsystem aus der aus 1,4-disubstituierten Cyclohexanringen, 4,4-disubstituierten Phenylcyclohexan- und Cyclohexylcyclohexansystemen, 2,5-disubstituierten Pyrimidin- und 1,3-Dioxanringen, 2,6-disubstituiertem Naphthalin, Di- und Tetranydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C | -CH₂-CH₂- |
| | -CO-O- | -CH₂-O- |
| | -CO-S- | -CH₂-S- |
| | -CH=N- | -COO-Phe-COO- |

oder eine C-C-Einfachbindung,
Y Chlor, oder -CN, und
R⁶ und R⁷ Alkyl, Alkoxy, Alkanoyloxy oder Alkoxycarbonyloxy mit bis zu 8 Kohlenstoffatomen, oder einer dieser Reste auch CN, NC, NO₂, CF₃, F, Cl oder Br bedeuten, sowie bevorzugte Tolane der Formel I.

Die Erfindung betrifft auch die Verwendung dieser Tolane als Komponenten flüssigkristalliner Phasen für elektro-optische Anzeigeelemente basierend auf dem ECB-Effekt.

Der Einfachheit halber bedeuten im folgenden Phe eine unsubstituierte 1,4-Phenylengruppe, PheX eine ein- oder mehrfach substituierte 1,4-Phenylengruppe (X bedeutet Halogen und/oder Nitril), Cyc eine trans-1,4-Cyclohexylengruppe, Dio eine 1,3-Dioxan-2,5-diylgruppe, Pyd eine Pyridin-2,5-diylgruppe, Pyr eine Pyrimidin-2,5-diylgruppe, Dit eine 1,3-Dithian-2,5-diylgruppe und Bco eine 1,4-Bicyclo(2.2.2)octylengruppe.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Verbindungen der Formel I sind vorzugsweise auch geeignet für die Verwendung als Komponenten in flüssigkristallinen Phasen für Displays, die auf dem ECB-Effekt beruhen.

Ähnliche Verbindungen sind z.B. bekannt aus GB 2155 465 A oder FR 2234 261, sowie aus Mol. Cryst. and Liq. Cryst. 62, 127 (1980); Chem. Abs., 87, No. 166967 s (1977); EP-A-0 255 700; EP-A-0 276 067; DE-A-25 04 641 und WO-A-88/02130.

Der Erfindung lag die Aufgabe zugrunde, neue stabile flüssigkristalline oder mesogene Verbindungen aufzufinden, die als Komponenten flüssigkristalliner Phasen geeignet sind. Diese Aufgabe wurde durch die Bereitstellung der Verbindungen der Formel I gelöst.

Es wurde gefunden, daß die Verbindungen der Formel I als Komponenten flüssigkristalliner Phasen vorzuglich geeignet sind. Insbesondere sind mit ihrer Hilfe stabile flüssigkristalline Phasen mit relativ großer optischer Anisotropie und ausgeprägt negativer dielektrischer Anisotropie herstellbar. Daher sind die Substanzen der Formel I bevorzugt für die Verwendung in Mischungen für ECB-Effekte geeignet.

Der ECB-Effekt (electrically controlled birefringence) oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys.Lett. 19 (1971), 3912). Es folgten Arbeiten von J.F. Kahn (Appl.Phys.Lett. 20 (1972), 1193) und G. Labrunie und J. Robert (J.Appl.Phys. 44 (1973), 4869).

Die Arbeiten von J. Robert und F. Clerc (SID 80 Digest Techn.Papers (1980), 30), J. Duchene (Displays 7 (1986), 3) und H. Schad (SID 82 Digest Techn. Papers (1982), 244) haben gezeigt, daß flüssigkristalline Phasen hohe Werte für das Verhältnis der elastischen Konstanten K₃/K₁, hohe Werte für die optische Anisotropie Δn und negative Werte für die dielektrische Anisotropie Δε aufweisen müssen, um für hochinformative Anzeigeelement basierend auf dem ECB-Effekt eingesetzt werden können.

Auf dem ECB-Effekt basierende elektrooptische Anzeigeelemente weisen eine homöotrope Randorientierung auf, d.h. die flüssigkristalline Phase hat eine negative dielektrische Anisotropie.

Für die technische Anwendung dieses Effektes in elektro-optischen Anzeigeelementen werden FK-Phasen benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren FK-Phasen eine flüssigkristalline Mesophase in einem geeigneten Temperaturbereich und eine niedrige Viskosität gefordert.

In keiner der bisher bekannten Reihen von Verbindungen mit flüssigkristalliner Mesophase gibt es eine Einzelverbindung, die allen diesen Erfordernissen entspricht. Es werden daher in der Regel Mischungen von zwei bis 25, vorzugsweise drei bis 18, Verbindungen hergestellt, um als FK-Phasen verwendbare Substanzen zu erhalten. Optimale Phasen konnten jedoch auf diese Weise nicht leicht hergestellt werden, da bisher keine Flüssigkristallmaterialien mit deutlich negativer dielektrischer Anisotropie und ausreichender Langzeitstabilität zur Verfügung standen.

Es besteht somit noch ein großer Bedarf an flüssigkristallinen Phasen mit günstigen Mesobereichen, hohen Werten für K₃/K₁, hoher optischer Anisotropie Δn, negativer dielektrischer Anisotropie Δε und hoher Langzeitstabilität.

Überraschend zeigte sich, daß der Zusatz von Verbindungen der Formel I flüssigkristalline Phasen liefert, die alle oben genannten Kriterien hervorragend erfüllen.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu optimieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit flüssigkristalline Phasen enthaltend Verbindungen der Formel I, bevorzugte Tolane der Formel I sowie die Verwendung dieser Verbindungen als Komponenten flüssigkristalliner Phasen und als Komponenten flüssigkristalliner Phasen für elektrooptische Anzeigeelemente basierend auf dem ECB-Effekt. Weiterhin sind Gegenstand der Erfindung flüssigkristalline Phasen sowie flüssigkristalline Phasen für elektrooptische Anzeigeelemente basierend auf dem ECB-Effekt mit einem Gehalt an mindestens einer Verbindung der Formel I sowie Flüssigkristall-Anzeigeelemente, insbesondere elektro-optische Anzeigeelemente, die derartige Phasen enthalten.

Vor- und nachstehen haben R¹, A¹, Z¹, m, A³, A⁴, A², n und R² die angegebene Bedeutung, sofern nicht ausdrücklich etwas anderes angegeben ist.

Die Verbindungen der Formel I umfassen dementsprechend Verbindungen der Teilformeln Ia (mit zwei Ringen), Ib und Ic (mit drei Ringen)

R¹-A³-C≡C-A⁴-R² Ia

R¹-A¹-A³-C≡C-A⁴-R² Ib

R¹-A¹-Z¹-A³-C≡C-A⁴-R² Ic

Die bevorzugten Verbindungen der Teilformel Ia umfassen solche der Teilformeln Iaa bis Iac:

R¹-Phe-C≡C-Phe-R² Iaa

R¹-Phe-C≡C-PheX-R² Iab

R¹-PheX-C≡C-PheX-R² Iac

Darunter sind diejenigen der Formeln Iaa und Iab besonders bevorzugt.

Die bevorzugten Verbindungen der Teilformeln Ib und Ic umfassen solche der Teilformeln I1 bis I2:

R¹-Phe-Phe-C≡C-Phe-R² I1

R¹-Phe-Phe-C≡C-PheX-R² I2

R¹-Phe-PheX-C≡C-Phe-R² I3

R¹-Phe-PheX-C≡C-PheX-R² I4

R¹-Cyc-Phe-C≡C-Phe-R² I5

R¹-Cyc-Phe-C≡C-PheX-R² I6

R¹-Cyc-PheX-C≡C-Phe-R² I7

R¹-A¹-CH₂CH₂-Phe-C≡C-Phe-R² I18

R¹-A¹-CH₂CH₂-Phe-C≡C-PheX-R² I22

Darunter sind diejenigen der Formeln I1, I2, I5, I6, I18, und I22 besonders bevorzugt. Besonders bevorzugt sind die Verbindungen der Teilformel I5.

In den Verbindungen der vor- und nachstehenden Formeln bedeuten R¹ und R² vorzugsweise Alkyl, Alkoxy oder eine andere Oxaalkyl- oder Dioxaalkylgruppe. Ferner sind Verbindungen der Formel I bevorzugt, in denen einer der Reste R¹ und R² Halogen, -OCF₃ oder -CF₃ ist.

m bedeutet 0 oder 1, wobei m vorzugsweise 0 ist.

A³ und A⁴ sind bevorzugt unsubstituiertes 1,4-Phenylen. Falls A³ und A⁴ substituiertes 1,4-Phenylen bedeuten, so ist es eine Substitution durch Fluor. Der Substituent steht bei einer Monosubstitution vorzugsweise in o-Stellung zu R² bzw. R¹. Der laterale Substituent kann aber auch in o-Stellung zur -C≡C-Dreifachbindung stehen.

Falls R¹ und/oder R² Alkylreste bedeuten, in denen auch eine ("Alkoxy" bzw. "Oxaalkyl") oder zwei ("Alkoxyalkoxy" bzw. "Dioxaalkyl") nicht benachbarte CH₂-Gruppen durch O-Atome ersetzt sein können, so können sie geradkettig oder verzweigt sein. Vorzugsweise sind sie geradkettig, haben 1, 2, 3, 4, 5, 6 oder 7 C-Atome und bedeuten demnach bevorzugt Methyl, Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy, Tetradecoxy, Pentadecoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formel I mit verzweigten Flügelgruppen R¹ oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verzweigte Gruppen dieser Art enthalten in der Regel nicht mehr als eine Kettenverzweigung. Bevorzugte verzweigte Rest sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy (= 2-Octyloxy), 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Methyl-3-oxapentyl, 2-Methyl-3-oxahexyl.

Bei Verbindungen mit verzweigten Flügelgruppen umfaßt Formel I sowohl die optischen Antipoden als auch Racemate sowie deren Gemische.

Unter den Verbindungen der Formel I und deren Unterformeln sind diejenigen bevorzugt, in denen mindestens einer der darin enthaltenen Reste eine der angegebenen bevorzugten Bedeutungen hat.

Besonders bevorzugte Verbindungen der Formel I sind diejenigen, in denen mindestens eine der Gruppen A³ und A⁴ eine 2,3-Diflour-1,4-phenylengruppe darstellt.

Im folgenden sind beispielhaft bevorzugte Vertreter von Verbindungen der Formel I mit einer 2,3-Difluor-1,4-phenylengruppe aufgezeigt (Teilformeln 1-9b): 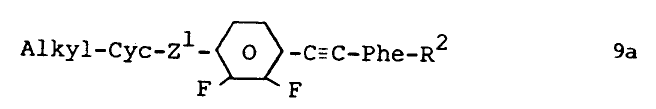 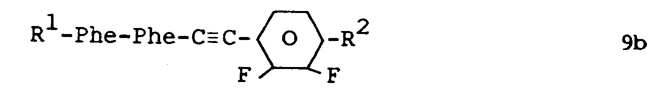
Für m = 0 sind von den Verbindungen der Formel I folgende Substanzen der Teilformeln 10-14 besonders bevorzugt:

Alkoxy-Phe-C≡C-Phe-F 10

Alkoxyalkoxy-Phe-C≡C-Phe-Alkyl 13

Alkoxyalkoxy-Phe-C≡C-Phe-Halogen 14

Eine kleinere Gruppe von besonders bevorzugten Verbindungen der Formel I für m ≠ 0 sind solche der Teilformeln 21-28:

Alkyl-Cyc-Phe-C≡C-Phe-Alkoxy 21

Alkyl-Cyc-CH₂CH₂-Phe-C≡C-Phe-Alkyl 22

Alkyl-Cyc-CH₂CH₂-Phe-C≡C-Phe-Alkoxy 23

Alkyl-Phe-Phe-C≡C-PheF-Halogen 24

Alkyl-Cyc-Phe-C≡C-PheF-Alkoxy 26

Alkyl-Phe-CH₂CH₂-Phe-C≡C-Phe-Alkyl 27

Alkyl-Phe-Phex-C≡C-Phe-Alkoxy 28

Besonders bevorzugt sind auch Verbindungen der Formel I, worin einer der Reste R¹ und R² eine -OCF₃ oder Trifluormethyl bedeutet. Insbesondere bevorzugt sind dabei beispielsweise Verbindungen der Teilformeln Iga bis Igk:

Alkyl-Cyc-Phe-C≡C-Phe-CF₃ Iga

Alkyl-Phe-Phe-C≡C-Phe-CF₃ Igc

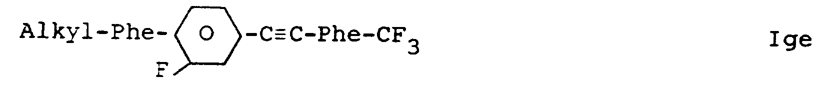

R¹-Phe-C≡C-Phe-CF₃ Igg

R¹-PheX-C≡C-Phe-CF₃ Igi

R¹-Phe-C≡C-Phe-OCF₃ Igj

R¹-Cyc-Phe-C≡C-Phe-OCF₃ Igk

Bevorzugte Verbindungen der Formel I sind diejenigen der Teilformel II 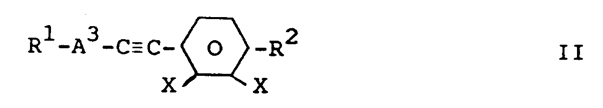
worin
- X: F bedeutet,
- R¹ und R²: die in Formel I angegebene Bedeutung haben,
- A³: unsubstituiertes oder ein- oder mehrfach durch Fluor substituiertes 1,4-Phenylen ist,
wobei vorzugsweise R¹ eine Alkyl- und R² eine Alkoxygruppe ist.

Bevorzugt sind auch die Verbindungen der Teilformel IIa

R¹-A³-C≡C-A⁴-R² IIa

worin
- A³ und A⁴: jeweils unsubstituiertes 1,4-Phenylen,
- R¹: Alkoxy mit bis zu 14 C-Atomen in der Alkylgruppe,
- R²: Fluor oder
einer der Reste R¹ und R² eine Alkylgruppe bedeutet, worin mindestens zwei nicht benachbarte CH₂-Gruppen durch -O- ersetzt sind.

Bevorzugte Verbindungen der Formel I sind auch diejenigen der Teilformel III 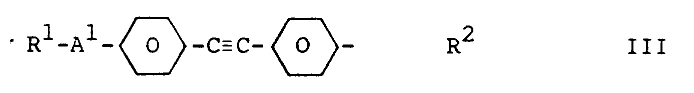
worin
- einer der Reste R¹ und R²: eine Alkoxygruppe mit bis zu 14 C-Atomen in der Alkylgruppe,
- der andere der Reste R¹ und R²: Halogen oder Alkyl mit bis zu 15 C-Atomen
bedeutet, und
- A¹: 1,4-Phenylen oder trans-1,4-Cyclohexylen, bedeutet.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Die Stilbene können hergestellt werden durch Umsetzung eines 4-substituierten Benzaldehyds mit einem entsprechenden Phosphorylid nach Wittig oder durch Umsetzung von einem 4-substituierten Phenylethylen mit einem entsprechenden Brombenzolderivat nach Heck.

Eine weitere Möglichkeit zur Herstellung der C-C-Dreifachbindung besteht darin, eine Verbindung, die sonst der Formel I entspricht, aber an Stelle der -C≡C-Bindung eine -CH₂-CO-Gruppe enthält, entweder mit einem anorganischen Säurechlorid umzusetzen, und die dann entstandene Gruppe -CH₂-CCl₂- in Gegenwart einer Base zu dehydrohalogenieren, oder mit Semicarbazid und Selendioxid umzusetzen und anschließend in Gegenwart von Methyllithium unter Erwärmen in die Dreifachbindung zu überführen.

Ferner besteht die Möglichkeit, ein entsprechendes Benzilderivat mit Hydrazin und anschließend mit HgO in das Tolan umzuwandeln.

Verbindungen der Formel I können auch hergestellt werden über die Kopplung von Alkinyl-Zink-Verbindungen mit Arylhalogeniden analog dem von A.O. King, E. Negishi, F.J. Villani und A. Silveira in J.Org.Chem. 43 (1978) 358 beschriebenen Verfahren.

Verbindungen der Formel I können auch über die Fritsch-Buttenberg-Wiechell-Umlagerung (Ann. 279, 319, 327, 332, 1894) hergestellt werden, bei der 1,1-Diaryl-2-halogenethylene umgelagert werden zu Diarylacetylenen in Gegenwart starker Basen.

Verbindungen der Formel I können weiterhin hergestellt werden aus 4-substituierten Phenylacetylenen und Arylhalogeniden in Gegenwart eines Palladiumkatalysators, z.B. Bis(triphenylphosphin)-palladium(II)-chlorid, und Kupfer(I)-jodid (beschrieben in Synthesis (1980) 627 oder Tetrahedron Letters 27 (1986) 1171).

Die erfindungsgemäßen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Di-phenylethane, 1,2-Dicyclohexylethane, 1-Phenyl-2-cyclohexylethane, gegebenenfalls halogenierten Stilbene, Benzylphenylether und substituierten Zimtsäuren.

Die wichtigsten als Bestandteile derartiger flüssigkristalliner Phasen in Frage kommenden Verbindungen lassen sich durch die Formel V charakterisieren,

R⁶-L-G-E-R⁷ V

worin L und E je ein carbo- oder heterocyclisches Ringsystem aus der aus 1,4-disubstituierten Cyclohexanringen, 4,4-disubstituierten Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CY- | -CH=N(O)- |
| | -C≡C | -CH₂-CH₂- |
| | -CO-O- | -CH₂-O- |
| | -CO-S- | -CH₂-S- |
| | -CH=N- | -COO-Phe-COO- |

oder eine C-C-Einfachbindung,
Y Chlor, oder -CN, und
R⁶ und R⁷ Alkyl, Alkoxy, Alkanoyloxy oder Alkoxycarbonyloxy mit bis zu 8 Kohlenstoffatomen, oder einer dieser Reste auch CN, NC, NO₂, CF₃, F, Cl oder Br bedeuten.

Bei den meisten dieser Verbindungen sind R⁶ und R⁷ voneinander verschieden, wobei einer dieser Reste meist eine Alkyl- oder Alkoxygruppe ist. Auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99 vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Verbindungen der Formel I können auch als Komponenten smektischer oder chiral getilteter smektischer flüssigkristalliner Phasen verwendet werden. Diese Phasen sind bevorzugt chiral getiltete smektische flüssigkristalline Phasen, deren achirale Basismischung neben Verbindungen der Formel I mindestens eine andere Komponente mit negativer oder betragsmäßig kleiner positiver dielektrischen Anisotropie enthält. Diese weitere(n) Komponente(n) der achiralen Basismischung kann (können) zu 1 bis 50 %, vorzugsweise 10 bis 25 %, der Basismischung ausmachen.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vg. z.B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die erfindungsgemäßen Phasen für elektrooptische Anzeigeelemente basierend auf dem ECB-Effekt enthalten vorzugsweise mindestens 7 % von Verbindungen der Formel I, insbesondere bevorzugt 7 bis 30 % von Verbindungen der Formel I.

Die folgenden Beispiele sollen die Erfindung erläutern, ohne sie zu begrenzen. Vor- und nachstehend bedeuten Prozentangaben Gewichtsprozent; alle Temperaturen sind in Grad Celsius angegeben.

"Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

F. bedeutet Schmelzpunkt und K. bedeutet Klärpunkt.

### Beispiel 1

Zu einem Gemisch aus 0,01 mol 4-Ethoxyjodbenzol, 0,01 mol 4-(trans-4-Propylcyclohexyl)-phenylacetylen (herstellbar z.B. nach Smith, Hoehn, Am. Soc. 63 (1941) 1175) und 40 ml Triethylamin gibt man bei Raumtemperatur 0,2 mmol Bis(triphenylphosphin)-palladium(II)-chlorid und 0,1 mmol Kupfer(I)-jodid und rührt 12 Stunden. Die Reaktion läßt sich mit Hilfe der Dünnschichtchromatographie verfolgen. Nach beendeter Reaktion wird die Suspension filtriert und das Filtrat eingedampft. Nach Reinigung durch Chromatographie und/oder Kristallisation erhält man 4-Ethoxy-4'-(trans-propylcyclohexyl)-tolan mit F. = 110° und K. = 253°.

Analog werden hergestellt:
4-Methoxy-4'-(trans-4-propylcyclohexyl)-tolan, F. 99°, K. 245°
4-Propyloxy-4'-(trans-4-propylcyclohexyl)-tolan, F. 97°, K. 236°
4-Butyloxy-4'-(trans-4-propylcyclohexyl)-tolan
4-Pentyloxy-4'-(trans-4-propylcyclohexyl)-tolan
4-Methoxy-4'-(trans-4-methylcyclohexyl)-tolan
4-Ethoxy-4'-(trans-4-methylcyclohexyl)-tolan
4-Propyloxy-4'-(trans-4-methylcyclohexyl)-tolan
4-Butyloxy-4'-(trans-4-methylcyclohexyl)-tolan
4-Pentyloxy-4'-(trans-4-methylcyclohexyl)-tolan
4-Methoxy-4'-(trans-4-ethylcyclohexyl)-tolan
4-Ethoxy-4'-(trans-4-ethylcyclohexyl)-tolan
4-Propyloxy-4'-(trans-4-ethylcyclohexyl)-tolan
4-Butyloxy-4'-(trans-4-ethylcyclohexyl)-tolan
4-Pentyloxy-4'-(trans-4-ethylcyclohexyl)-tolan
4-Methoxy-4'-(trans-4-butylcyclohexyl)-tolan
4-Ethoxy-4'-(trans-4-butylcyclohexyl)-tolan
4-Propyloxy-4'-(trans-4-butylcyclohexyl)-tolan
4-Butyloxy-4'-(trans-4-butylcyclohexyl)-tolan
4-Pentyloxy-4'-(trans-4-butylcyclohexyl)-tolan
4-Methoxy-4'-(trans-4-pentylcyclohexyl)-tolan
4-Ethoxy-4'-(trans-4-pentylcyclohexyl)-tolan
4-Propyloxy-4'-(trans-4-pentylcyclohexyl)-tolan
4-Butyloxy-4'-(trans-4-pentylcyclohexyl)-tolan
4-Pentyloxy-4'-(trans-4-pentylcyclohexyl)-tolan
4-Methoxy-4'-(trans-4-heptylcyclohexyl)-tolan
4-Ethoxy-4'-(trans-4-heptylcyclohexyl)-tolan
4-Propyloxy-4'-(trans-4-heptylcyclohexyl)-tolan
4-Butyloxy-4'-(trans-4-heptylcyclohexyl)-tolan
4-Pentyloxy-4'-(trans-4-heptylcyclohexyl)-tolan
4-Methoxy-4'-(trans-4-methylcyclohexylethyl)-tolan
4-Ethoxy-4'-(trans-4-methylcyclohexylethyl)-tolan
4-Propyloxy-4'-(trans-4-methylcyclohexylethyl)-tolan
4-Butyloxy-4'-(trans-4-methylcyclohexylethyl)-tolan
4-Pentyloxy-4'-(trans-4-methylcyclohexylethyl)-tolan
4-Methoxy-4'-(trans-4-ethylcyclohexylethyl)-tolan
4-Ethoxy-4'-(trans-4-ethylcyclohexylethyl)-tolan
4-Propyloxy-4'-(trans-4-ethylcyclohexylethyl)-tolan
4-Butyloxy-4'-(trans-4-ethylcyclohexylethyl)-tolan
4-Pentyloxy-4'-(trans-4-ethylcyclohexylethyl)-tolan
4-Methoxy-4'-(trans-4-propylcyclohexylethyl)-tolan
4-Ethoxy-4'-(trans-4-propylcyclohexylethyl)-tolan
4-Propyloxy-4'-(trans-4-propylcyclohexylethyl)-tolan
4-Butyloxy-4'-(trans-4-propylcyclohexylethyl)-tolan
4-Pentyloxy-4'-(trans-4-propylcyclohexylethyl)-tolan
4-Methoxy-4'-(trans-4-pentylcyclohexylethyl)-tolan
4-Ethoxy-4'-(trans-4-pentylcyclohexylethyl)-tolan
4-Propyloxy-4'-(trans-4-pentylcyclohexylethyl)-tolan
4-Butyloxy-4'-(trans-4-pentylcyclohexylethyl)-tolan
4-Pentyloxy-4'-(trans-4-pentylcyclohexylethyl)-tolan
4-Methyl-4'-(trans-4-propylcyclohexylethyl)-tolan
4-Ethyl-4'-(trans-4-propylcyclohexylethyl)-tolan
4-Propyl-4'-(trans-4-propylcyclohexylethyl)-tolan
4-Butyl-4'-(trans-4-propylcyclohexylethyl)-tolan
4-Pentyl-4'-(trans-4-propylcyclohexylethyl)-tolan
4-Methyl-4'-(trans-4-pentylcyclohexylethyl)-tolan
4-Ethyl-4'-(trans-4-pentylcyclohexylethyl)-tolan
4-Propyl-4'-(trans-4-pentylcyclohexylethyl)-tolan
4-Butyl-4'-(trans-4-pentylcyclohexylethyl)-tolan
4-Pentyl-4'-(trans-4-pentylcyclohexylethyl)-tolan
3,4-Difluor-4'-(-4-methylphenyl)-tolan
3,4-Difluor-4'-(4-ethylphenyl)-tolan
3,4-Difluor-4'-(4-propylphenyl)-tolan
3,4-Difluor-4'-(4-butylphenyl)-tolan
3,4-Difluor-4'-(4-pentylphenyl)-tolan
3-Fluor-4-methoxy-4'-(trans-4-propylcyclohexyl)-tolan, F. 107°, K. 208°
3-Fluor-4-ethoxy-4'-(trans-4-propylcyclohexyl)-tolan
3-Fluor-4-propyloxy-4'-(trans-4-propylcyclohexyl)-tolan
3-Fluor-4-butyloxy-4'-(trans-4-propylcyclohexyl)-tolan
3-Fluor-4-pentyloxy-4'-(trans-4-propylcyclohexyl)-tolan
3-Fluor-4-methoxy-4'-(trans-4-pentylcyclohexyl)-tolan
3-Fluor-4-ethoxy-4'-(trans-4-pentylcyclohexyl)-tolan
3-Fluor-4-propyloxy-4'-(trans-4-pentylcyclohexyl)-tolan
3-Fluor-4-butyloxy-4'-(trans-4-pentylcyclohexyl)-tolan
3-Fluor-4-pentyloxy-4'-(trans-4-pentylcyclohexyl)-tolan
4-Methyl-4'-(4-propylphenylethyl)-tolan
4-Ethyl-4'-(4-propylphenylethyl)-tolan
4-Propyl-4'-(4-propylphenylethyl)-tolan
4-Butyl-4'-(4-propylphenylethyl)-tolan
4-Pentyl-4'-(4-propylphenylethyl)-tolan
4-Methyl-4'-(4-butylphenylethyl)-tolan
4-Ethyl-4'-(4-butylphenyletnyl)-tolan
4-Propyl-4'-(4-butylphenylethyl)-tolan
4-Butyl-4'-(4-butylphenylethyl)-tolan
4-Pentyl-4'-(4-butylphenylethyl)-tolan
4-Methyl-4'-(4-ethylphenylethyl)-tolan
4-Ethyl-4'-(4-ethylphenylethyl)-tolan
4-Propyl-4'-(4-ethylphenylethyl)-tolan
4-Butyl-4'-(4-ethylphenylethyl)-tolan
4-Pentyl-4'-(4-ethylphenylethyl)-tolan
4-Methyl-4'-(4-pentylphenylethyl)-tolan
4-Ethyl-4'-(4-pentylphenylethyl)-tolan
4-Propyl-4'-(4-pentylphenylethyl)-tolan
4-Butyl-4'-(4-pentylphenylethyl)-tolan
4-Pentyl-4'-(4-pentylphenylethyl)-tolan
4-Fluor-4'-(4-ethoxyphenyl)-tolan
4-Fluor-4'-(4-propyloxyphenyl)-tolan
4-Fluor-4'-(4-butyloxyphenyl)-tolan
4-Fluor-4'-(4-pentyloxyphenyl)-tolan
4-Chlor-4'-(4-methoxyphenyl)-tolan
4-Chlor-4'-(4-ethoxyphenyl)-tolan
4-Chlor-4'-(4-propyloxyphenyl)-tolan
4-Chlor-4'-(4-butyloxyphenyl)-tolan
4-Chlor-4'-(4-pentyloxyphenyl)-tolan
4-Methoxy-3'-fluor-4'-(4-ethylphenyl)-tolan
4-Methoxy-3'-fluor-4'-(4-propylphenyl)-tolan
4-Methoxy-3'-fluor-4'-(4-butylphenyl)-tolan
4-Methoxy-3'-fluor-4'-(4-pentylphenyl)-tolan
4-Methoxy-3'-fluor-4'-(4-hexylphenyl)-tolan
4-Methoxy-3'-fluor-4'-(4-heptylphenyl)-tolan
4-Ethoxy-3'-fluor-4'-(4-ethylphenyl)-tolan
4-Ethoxy-3'-fluor-4'-(4-propylphenyl)-tolan
4-Ethoxy-3'-fluor-4'-(4-butylphenyl)-tolan, F. = 88°, K. = 208°
4-Ethoxy-3'-fluor-4'-(4-pentylphenyl)-tolan
4-Ethoxy-3'-fluor-4'-(4-hexylphenyl)-tolan
4-Ethoxy-3'-fluor-4'-(4-heptylphenyl)-tolan
4-Propoxy-3'-fluor-4'-(4-ethylphenyl)-tolan
4-Propoxy-3'-fluor-4'-(4-propylphenyl)-tolan
4-Propoxy-3'-fluor-4'-(4-butylphenyl)-tolan
4-Propoxy-3'-fluor-4'-(4-pentylphenyl)-tolan
4-Propoxy-3'-fluor-4'-(4-hexylphenyl)-tolan
4-Propoxy-3'-fluor-4'-(4-heptylphenyl)-tolan
4-Butoxy-3'-fluor-4'-(4-ethylphenyl)-tolan
4-Butoxy-3'-fluor-4'-(4-propylphenyl)-tolan
4-Butoxy-3'-fluor-4'-(4-butylphenyl)-tolan
4-Butoxy-3'-fluor-4'-(4-pentylphenyl)-tolan
4-Butoxy-3'-fluor-4'-(4-hexylphenyl)-tolan
4-Butoxy-3'-fluor-4'-(4-heptylphenyl)-tolan
4-Pentyloxy-3'-fluor-4'-(4-ethylphenyl)-tolan
4-Pentyloxy-3'-fluor-4'-(4-propylphenyl)-tolan
4-Pentyloxy-3'-fluor-4'-(4-butylphenyl)-tolan
4-Pentyloxy-3'-fluor-4'-(4-pentylphenyl)-tolan
4-Pentyloxy-3'-fluor-4'-(4-hexylphenyl)-tolan
4-Pentyloxy-3'-fluor-4'-(4-heptylphenyl)-tolan
4-(4-Methoxyphenyl)-4'-(trans-4-methylcyclohexyl)-tolan
4-(4-Ethoxyphenyl)-4'-(trans-4-methylcyclohexyl)-tolan
4-(4-Propyloxyphenyl)-4'-(trans-4-methylcyclohexyl)-tolan
4-(4-Butyloxyphenyl)-4'-(trans-4-methylcyclohexyl)-tolan
4-(4-Pentyloxyphenyl)-4'-(trans-4-methylcyclohexyl)-tolan
4-(4-Methoxyphenyl)-4'-(trans-4-ethylcyclohexyl)-tolan
4-(4-Ethoxyphenyl)-4'-(trans-4-ethylcyclohexyl)-tolan
4-(4-Propyloxyphenyl)-4'-(trans-4-ethylcyclohexyl)-tolan
4-(4-Butyloxyphenyl)-4'-(trans-4-ethylcyclohexyl)-tolan
4-(4-Pentyloxyphenyl)-4'-(trans-4-ethylcyclohexyl)-tolan
4-(4-Methoxyphenyl)-4'-(trans-4-propylcyclohexyl)-tolan
4-(4-Ethoxyphenyl)-4'-(trans-4-propylcyclohexyl)-tolan
4-(4-Propyloxyphenyl)-4'-(trans-4-propylcyclohexyl)-tolan
4-(4-Butyloxyphenyl)-4'-(trans-4-propylcyclohexyl)-tolan
4-(4-Pentyloxyphenyl)-4'-(trans-4-propylcyclohexyl)-tolan
4-(4-Methoxyphenyl)-4'-(trans-4-butylcyclohexyl)-tolan
4-(4-Ethoxyphenyl)-4'-(trans-4-butylcyclohexyl)-tolan
4-(4-Propyloxyphenyl)-4'-(trans-4-butylcyclohexyl)-tolan
4-(4-Butyloxyphenyl)-4'-(trans-4-butylcyclohexyl)-tolan
4-(4-Pentyloxyphenyl)-4'-(trans-4-butylcyclohexyl)-tolan.

### Beispiel 2

Analog Beispiel 1 erhält man aus 4-Heptyloxyphenylacetylen und 4-Fluorjodbenzol das entsprechende 4-Fluor-4'-heptyloxy-tolan mit F. 63° und K. 52.3°.

Analog werden hergestellt:
4-Fluor-4'-ethoxy-tolan
4-Fluor-4'-propyloxy-tolan
4-Fluor-4'-butyloxy-tolan
4-Fluor-4'-pentyloxy-tolan
4-Methyl-4'-methoxymethoxy-tolan
4-Ethyl-4'-methoxymethoxy-tolan
4-Propyl-4'-methoxymethoxy-tolan
4-Butyl-4'-methoxymethoxy-tolan
4-Pentyl-4'-methoxymethoxy-tolan
4-Hexyl-4'-methoxymethoxy-tolan
4-Heptyl-4'-methoxymethoxy-tolan
4-Methyl-4'-(2-methoxyethoxy)-tolan
4-Ethyl-4'-(2-methoxyethoxy)-tolan
4-Propyl-4'-(2-methoxyethoxy)-tolan
4-Butyl-4'-(2-methoxyethoxy)-tolan
4-Pentyl-4'-(2-methoxyethoxy)-tolan
4-Hexyl-4'-(2-methoxyethoxy)-tolan
4-Heptyl-4'-(2-methoxyethoxy)-tolan
4-Methyl-4'-ethoxymethoxy-tolan
4-Ethyl-4'-ethoxymethoxy-tolan
4-Propyl-4'-ethoxymethoxy-tolan
4-Butyl-4'-ethoxymethoxy-tolan
4-Pentyl-4'-ethoxymethoxy-tolan
4-Hexyl-4'-ethoxymethoxy-tolan
4-Heptyl-4'-ethoxymethoxy-tolan
4-Methyl-4'-(2-ethoxyethoxy)-tolan
4-Ethyl-4'-(2-ethoxyethoxy)-tolan
4-Propyl-4'-(2-ethoxyethoxy)-tolan
4-Butyl-4'-(2-ethoxyethoxy)-tolan
4-Pentyl-4'-(2-ethoxyethoxy)-tolan
4-Hexyl-4'-(2-ethoxyethoxy)-tolan
4-Heptyl-4'-(2-ethoxyethoxy)-tolan
4-Methyl-4'-propyloxymethoxy-tolan
4-Ethyl-4'-propyloxymethoxy-tolan
4-Propyl-4'-propyloxymethoxy-tolan
4-Butyl-4'-propyloxymethoxy-tolan
4-Pentyl-4'-propyloxymethoxy-tolan
4-Hexyl-4'-propyloxymethoxy-tolan
4-Heptyl-4'-propyloxymethoxy-tolan
4-Methyl-4'-(2-propyloxyethoxy)-tolan
4-Ethyl-4'-(2-propyloxyethoxy)-tolan
4-Propyl-4'-(2-propyloxyethoxy)-tolan
4-Butyl-4'-(2-propyloxyethoxy)-tolan
4-Pentyl-4'-(2-propyloxyethoxy)-tolan
4-Hexyl-4'-(2-propyloxyethoxy)-tolan
4-Heptyl-4'-(2-propyloxyethoxy)-tolan
4-Fluor-4'-methoxymethoxy-tolan
4-Fluor-4'-(2-methoxyethoxy)-tolan
4-Chlor-4'-propyloxymethoxy-tolan
4-Chlor-4'-(2-methoxyethoxy)-tolan
4-Fluor-4'-(2-ethoxyethoxy)-tolan
4-Fluor-4'-(2-propyloxyethoxy)-tolan

### Beispiel 3

a) Ein Gemisch aus 0,1 mol 2,3-Difluorethoxybenzol, 0,1 mol Tetramethylethylendiamin (TMEDA) und 200 ml THF wird bei -70 ° bis -60 ° mit n-Butyllithium (0,105 mol) versetzt und ca. 4 Stunden bei -70 ° gerührt. Dann wird das Reaktionsgemisch bei dieser Temperatur mit 0,1 mol 4-(trans-4-Propylcyclohexyl)-acetophenon versetzt. Dann läßt man das Gemisch auf Raumtemperatur kommen und hydrolysiert mit ges. NH₄Cl-Lösung. Man trennt die organische Phase ab und arbeitet sie auf. Der Rückstand wird mit 2 g p-Toluolsulfonsäure und 200 ml Toluol am Wasserabscheider erhitzt. Nach Aufarbeitung und Umkristallisation erhält man 1-(2,3-Difluor-4-ethoxyphenyl)-1-[4-(trans-4-propylcyclohexyl)phenyl]-ethylen.
b) Zu einer Lösung von 0,065 mol des in a) hergestellten Ethylenderivats in 100 ml CH₂Cl₂ und 50 ml Acetonitril werden 0,065 mol Brom bei 0° zugegeben. Anschließend gibt man 10 g Triethylamin zu und arbeitet wie üblich auf.
   Der Rückstand wird in ca. 50 ml THF aufgenommen und bei -50° zu einer bei -30° hergestellten Lösung von Lithiumdiisopropylamid in THF (120 ml THF, 0,13 mol Diisopropylamin und 0,13 mol n-Butyllithium) gegeben. Man rührt über Nacht, wobei die Reaktionsmischung langsam auf Raumtemperatur kommt. Dann wird mit H₂O hydrolisiert , die organische Phase aufgearbeitet und man erhält nach Umkristallisation aus Aceton/Ethanol 2,3-Difluor-4-ethoxy-4'-(trans-4-propylcyclohexyl)-tolan mit F. = 87° und K. = 231°.

Analog werden hergestellt:
2,3-Difluor-4-ethoxy-4'-(trans-4-methylcyclohexyl)-tolan
2,3-Difluor-4-ethoxy-4'-(trans-4-ethylcyclohexyl)-tolan
2,3-Difluor-4-ethoxy-4'-(trans-4-butylcyclohexyl)-tolan
2,3-Difluor-4-ethoxy-4'-(trans-4-pentylcyclohexyl)-tolan
2,3-Difluor-4-etnoxy-4'-(trans-4-hexylcyclohexyl)-tolan
2,3-Difluor-4-ethoxy-4'-(trans-4-heptylcyclohexyl)-tolan
2,3-Difluor-4-methoxy-4'-(trans-4-methylcyclohexyl)-tolan
2,3-Difluor-4-methoxy-4'-(trans-4-ethylcyclohexyl)-tolan
2,3-Difluor-4-methoxy-4'-(trans-4-propylcyclohexyl)-tolan
2,3-Difluor-4-methoxy-4'-(trans-4-butylcyclohexyl)-tolan
2,3-Difluor-4-methoxy-4'-(trans-4-pentylcyclohexyl)-tolan
2,3-Difluor-4-methoxy-4'-(trans-4-hexylcyclohexyl)-tolan
2,3-Difluor-4-methoxy-4'-(trans-4-heptylcyclohexyl)-tolan
2,3-Difluor-4-propoxy-4'-(trans-4-methylcyclohexyl)-tolan
2,3-Difluor-4-propoxy-4'-(trans-4-ethylcyclohexyl)-tolan
2,3-Difluor-4-propoxy-4'-(trans-4-propylcyclohexyl)-tolan
2,3-Difluor-4-propoxy-4'-(trans-4-butylcyclohexyl)-tolan
2,3-Difluor-4-propoxy-4'-(trans-4-pentylcyclohexyl)-tolan
2,3-Difluor-4-propoxy-4'-(trans-4-hexylcyclohexyl)-tolan
2,3-Difluor-4-propoxy-4'-(trans-4-heptylcyclohexyl)-tolan
2,3-Difluor-4-butoxy-4'-(trans-4-methylcyclohexyl)-tolan
2,3-Difluor-4-butoxy-4'-(trans-4-ethylcyclohexyl)-tolan
2,3-Difluor-4-butoxy-4'-(trans-4-propylcyclohexyl)-tolan
2,3-Difluor-4-butoxy-4'-(trans-4-butylcyclohexyl)-tolan
2,3-Difluor-4-butoxy-4'-(trans-4-pentylcyclohexyl)-tolan
2,3-Difluor-4-butoxy-4'-(trans-4-hexylcyclohexyl)-tolan
2,3-Difluor-4-butoxy-4'-(trans-4-heptylcyclohexyl)-tolan
2,3-Difluor-4-pentyloxy-4'-(trans-4-methylcyclohexyl)-tolan
2,3-Difluor-4-pentyloxy-4'-(trans-4-ethylcyclohexyl)-tolan
2,3-Difluor-4-pentyloxy-4'-(trans-4-propylcyclohexyl)-tolan
2,3-Difluor-4-pentyloxy-4'-(trans-4-butylcyclohexyl)-tolan
2,3-Difluor-4-pentyloxy-4'-(trans-4-pentylcyclohexyl)-tolan
2,3-Difluor-4-pentyloxy-4'-(trans-4-hexylcyclohexyl)-tolan
2,3-Difluor-4-pentyloxy-4'-(trans-4-heptylcyclohexyl)-tolan
2,3-Difluor-4-pentyloxy-4'-(4-methylphenyl)-tolan
2,3-Difluor-4-pentyloxy-4'-(4-ethylphenyl)-tolan
2,3-Difluor-4-pentyloxy-4'-(4-propylphenyl)-tolan
2,3-Difluor-4-pentyloxy-4'-(4-butylphenyl)-tolan
2,3-Difluor-4-pentyloxy-4'-(4-pentylphenyl)-tolan
2,3-Difluor-4-pentyloxy-4'-(4-hexylphenyl)-tolan
2,3-Difluor-4-pentyloxy-4'-(4-heptylphenyl)-tolan
2,3-Difluor-4-butoxy-4'-(4-methylphenyl)-tolan
2,3-Difluor-4-butoxy-4'-(4-ethylphenyl)-tolan
2,3-Difluor-4-butoxy-4'-(4-propylphenyl)-tolan
2 3-Difluor-4-butoxy-4'-(4-butylphenyl)-tolan
2,3-Difluor-4-butoxy-4'-(4-pentylphenyl)-tolan
2,3-Difluor-4-butoxy-4'-(4-hexylphenyl)-tolan
2,3-Difluor-4-butoxy-4'-(4-heptylphenyl)-tolan
2,3-Difluor-4-propoxy-4'-(4-methylphenyl)-tolan
2,3-Difluor-4-propoxy-4'-(4-ethylphenyl)-tolan
2,3-Difluor-4-propoxy-4'-(4-propylphenyl)-tolan
2,3-Difluor-4-propoxy-4'-(4-butylphenyl)-tolan
2,3-Difluor-4-propoxy-4'-(4-pentylphenyl)-tolan
2,3-Difluor-4-propoxy-4'-(4-hexylphenyl)-tolan
2,3-Difluor-4-propoxy-4'-(4-heptylphenyl)-tolan
2,3-Difluor-4-ethoxy-4'-(4-methylphenyl)-tolan
2,3-Difluor-4-ethoxy-4'-(4-ethylphenyl)-tolan
2,3-Difluor-4-ethoxy-4'-(4-propylphenyl)-tolan
2,3-Difluor-4-ethoxy-4'-(4-butylphenyl)-tolan
2,3-Difluor-4-ethoxy-4'-(4-pentylphenyl)-tolan
2,3-Difluor-4-ethoxy-4'-(4-hexylphenyl)-tolan
2,3-Difluor-4-ethoxy-4'-(4-heptylphenyl)-tolan
2,3-Difluor-4-methoxy-4'-(4-methylphenyl)-tolan
2,3-Difluor-4-methoxy-4'-(4-ethylphenyl)-tolan
2,3-Difluor-4-methoxy-4'-(4-propylphenyl)-tolan
2,3-Difluor-4-methoxy-4'-(4-butylphenyl)-tolan
2,3-Difluor-4-methoxy-4'-(4-pentylphenyl)-tolan
2,3-Difluor-4-methoxy-4'-(4-hexylphenyl)-tolan
2,3-Difluor-4-methoxy-4'-(4-heptylphenyl)-tolan
2,3-Difluor-4-(4-methylphenyl)-4'-methyl-tolan
2,3-Difluor-4-(4-ethylphenyl)-4'-methyl-tolan
2,3-Difluor-4-(4-propylphenyl)-4'-methyl-tolan
2,3-Difluor-4-(4-butylphenyl)-4'-methyl-tolan
2,3-Difluor-4-(4-pentylphenyl)-4'-methyl-tolan
2,3-Difluor-4-(4-methylphenyl)-4'-ethyl-tolan
2,3-Difluor-4-(4-ethylphenyl)-4'-ethyl-tolan
2,3-Difluor-4-(4-propylphenyl)-4'-ethyl-tolan
2,3-Difluor-4-(4-butylphenyl)-4'-ethyl-tolan
2,3-Difluor-4-(4-pentylphenyl)-4'-ethyl-tolan
2,3-Difluor-4-(4-methylphenyl)-4'-propyl-tolan
2,3-Difluor-4-(4-ethylphenyl)-4'-propyl-tolan
2,3-Difluor-4-(4-propylphenyl)-4'-propyl-tolan
2,3-Difluor-4-(4-butylphenyl)-4'-propyl-tolan
2,3-Difluor-4-(4-pentylphenyl)-4'-propyl-tolan
2,3-Difluor-4-(4-methylphenyl)-4'-butyl-tolan
2,3-Difluor-4-(4-ethylphenyl)-4'-butyl-tolan
2,3-Difluor-4-(4-propylphenyl)-4'-butyl-tolan
2,3-Difluor-4-(4-butylphenyl)-4'-butyl-tolan
2,3-Difluor-4-(4-pentylphenyl)-4'-butyl-tolan
2,3-Difluor-4-(4-methylphenyl)-4'-pentyl-tolan
2,3-Difluor-4-(4-ethylphenyl)-4'-pentyl-tolan
2,3-Difluor-4-(4-propylphenyl)-4'-pentyl-tolan
2,3-Difluor-4-(4-butylphenyl)-4'-pentyl-tolan
2,3-Difluor-4-(4-pentylphenyl)-4'-pentyl-tolan
2,3-Difluor-4-(4-methylphenyl)-4'-fluor-tolan
2,3-Difluor-4-(4-ethylphenyl)-4'-fluor-tolan
2,3-Difluor-4-(4-propylphenyl)-4'-fluor-tolan
2,3-Difluor-4-(4-butylphenyl)-4'-fluor-tolan
2,3-Difluor-4-(4-pentylphenyl)-4'-fluor-tolan

### Beispiel 4

a) Aus 0,1 mol 2,3-Difluorethoxybenzol und 0,1 mol 4-Pentylacetophenon erhält man analog Beispiel 3a 1-(4-Pentylphenyl)-1-(2,3-difluor-4-ethoxyphenyl)-ethylen.
b) Analog Beispiel 3b erhält man aus 1-(4-Pentyl-phenyl)-1-(2,3-difluor-4-ethoxyphenyl)-ethylen nach Umkristallisation aus Methanol/Ethanol 2,3-Difluor-4-ethoxy-4'-pentyl-tolan mit F. = 57° und K. = 61°.

Analog werden hergestellt:
2,3-Difluor-4-ethoxy-4'-methyltolan
2,3-Difluor-4-ethoxy-4'-ethyltolan
2,3-Difluor-4-ethoxy-4'-propyltolan
2,3-Difluor-4-ethoxy-4'-butyltolan
2,3-Difluor-4-ethoxy-4'-hexyltolan
2,3-Difluor-4-ethoxy-4'-heptyltolan
2,3-Difluor-4-methoxy-4'-methyltolan
2,3-Difluor-4-methoxy-4'-ethyltolan
2,3-Difluor-4-methoxy-4'-propyltolan
2,3-Difluor-4-methoxy-4'-butyltolan
2,3-Difluor-4-methoxy-4'-pentyltolan
2,3-Difluor-4-methoxy-4'-hexyltolan
2,3-Difluor-4-methoxy-4'-heptyltolan
2,3-Difluor-4-propoxy-4'-methyltolan
2,3-Difluor-4-propoxy-4'-ethyltolan
2,3-Difluor-4-propoxy-4'-propyltolan
2,3-Difluor-4-propoxy-4'-butyltolan
2,3-Difluor-4-propoxy-4'-pentyltolan
2,3-Difluor-4-propoxy-4'-hexyltolan
2,3-Difluor-4-propoxy-4'-heptyltolan
2,3-Difluor-4-butoxy-4'-methyltolan
2,3-Difluor-4-butoxy-4'-ethyltolan
2,3-Difluor-4-butoxy-4'-propyltolan
2,3-Difluor-4-butoxy-4'-butyltolan
2,3-Difluor-4-butoxy-4'-pentyltolan
2,3-Difluor-4-butoxy-4'-hexyltolan
2,3-Difluor-4-butoxy-4'-heptyltolan
2,3-Difluor-4-pentyloxy-4'-methyltolan
2,3-Difluor-4-pentyloxy-4'-ethyltolan
2,3-Difluor-4-pentyloxy-4'-propyltolan
2,3-Difluor-4-pentyloxy-4'-butyltolan
2,3-Difluor-4-pentyloxy-4'-pentyltolan
2,3-Difluor-4-pentyloxy-4'-hexyltolan
2,3-Difluor-4-pentyloxy-4'-heptyltolan
2,3-Difluor-4-hexyloxy-4'-methyltolan
2,3-Difluor-4-hexyloxy-4'-ethyltolan
2,3-Difluor-4-hexyloxy-4'-propyltolan
2,3-Difluor-4-hexyloxy-4'-butyltolan
2,3-Difluor-4-hexyloxy-4'-pentyltolan
2,3-Difluor-4-hexyloxy-4'-hexyltolan
2,3-Difluor-4-hexyloxy-4'-heptyltolan
2,3-Difluor-4-ethyl-4'-methoxy-tolan
2,3-Difluor-4-propyl-4'-methoxy-tolan
2,3-Difluor-4-butyl-4'-methoxy-tolan
2,3-Difluor-4-pentyl-4'-methoxy-tolan
2,3-Difluor-4-ethyl-4'-ethoxy-tolan
2,3-Difluor-4-propyl-4'-ethoxy-tolan
2,3-Difluor-4-butyl-4'-ethoxy-tolan
2,3-Difluor-4-pentyl-4'-ethoxy-tolan
2,3-Difluor-4-ethyl-4'-propoxy-tolan
2,3-Difluor-4-propyl-4'-propoxy-tolan
2,3-Difluor-4-butyl-4'-propoxy-tolan
2,3-Difluor-4-pentyl-4'-propoxy-tolan
2,3-Difluor-4-ethyl-4'-butoxy-tolan
2,3-Difluor-4-propyl-4'-butoxy-tolan
2,3-Difluor-4-butyl-4'-butoxy-tolan
2,3-Difluor-4-pentyl-4'-butoxy-tolan
2,3-Difluor-4-ethyl-4'-pentoxy-tolan
2,3-Difluor-4-propyl-4'-pentoxy-tolan
2,3-Difluor-4-butyl-4'-penthoxy-tolan
2,3-Difluor-4-pentyl-4'-pentoxy-tolan
2,3-Difluor-4-(trans-4-ethylcyclohexylethylen)-4'-ethyl-tolan
2,3-Difluor-4-(trans-4-propylcyclohexylethylen)-4'-ethyl-tolan
2,3-Difluor-4-(trans-4-butylcyclohexylethylen)-4'-ethyl-tolan
2,3-Difluor-4-(trans-4-pentylcyclohexylethylen)-4'-ethyl-tolan
2,3-Difluor-4-(trans-4-ethylcyclohexylethylen)-4'-propyl-tolan
2,3-Difluor-4-(trans-4-propylcyclohexylethylen)-4'-propyl-tolan
2,3-Difluor-4-(trans-4-butylcyclohexylethylen)-4'-propyl-tolan
2,3-Difluor-4-(trans-4-pentylcyclohexylethylen)-4'-propyl-tolan
2,3-Difluor-4-(trans-4-ethylcyclohexylethylen)-4'-butyl-tolan
2,3-Difluor-4-(trans-4-propylcyclohexylethylen)-4'-butyl-tolan
2,3-Difluor-4-(trans-4-butylcyclohexylethylen)-4'-butyl-tolan
2,3-Difluor-4-(trans-4-pentylcyclohexylethylen)-4'-butyl-tolan
2,3-Difluor-4-(trans-4-ethylcyclohexylethylen)-4'-pentyl-tolan
2,3-Difluor-4-(trans-4-propylcyclohexylethylen)-4'-pentyl-tolan
2,3-Difluor-4-(trans-4-butylcyclohexylethylen)-4'-pentyl-tolan
2,3-Difluor-4-(trans-4-pentylcyclohexylethylen)-4'-pentyl-tolan
2,3-Difluor-4-(trans-4-ethylcyclohexylethylen)-4'-fluor-tolan
2,3-Difluor-4-(trans-4-propylcyclohexylethylen)-4'-fluor-tolan
2,3-Difluor-4-(trans-4-butylcyclohexylethylen)-4'-fluor-tolan
2,3-Difluor-4-(trans-4-pentylcyclohexylethylen)-4'-fluor-tolan
2,3-Difluor-4-(trans-4-ethylcyclohexylethylen)-4'-trifluormethyl-tolan
2,3-Difluor-4-(trans-4-propylcyclohexylethylen)-4'-trifluormethyl-tolan
2,3-Difluor-4-(trans-4-butylcyclohexylethylen)-4'-trifluormethyl-tolan
2,3-Difluor-4-(trans-4-pentylcyclohexylethylen)-4'-trifluormethyl-tolan

### Beispiel 5

Ein Gemisch aus 0,02 m 4-(trans-4-Propylcyclohexyl)phenylacetylen, 0,02 m 4-Trifluormethoxyiodbenzol und 50 ml Triethylamin wird bei Raumtemperatur mit 0,4 mmol Bis-(triphenylphosphin)-palladium(II)-chlorid und 0,2 mmol CuJ versetzt. Man rührt 2 Stunden bei Raumtemperatur, verdünnt dann das Reaktionsgemisch mit 100 ml Methyl-tert.butylether, saugt ab und dampft die Lösung ein. Nach Reinigung durch Chromatographie und/oder Kristallisation erhält man 4-Trifluormethoxy-4'-(trans-4-propylcyclohexyl)-tolan mit K 88° S_{B} 126° S_{A} 163° N 198° I.

Analog werden hergestellt:
4-Trifluormethoxy-4'-(trans-4-ethylcyclohexyl)-tolan
4-Trifluormethoxy-4'-(trans-4-butylcyclohexyl)-tolan
4-Trifluormethoxy-4'-(trans-4-pentylcyclohexyl)-tolan
4-Trifluormethoxy-4'-(trans-4-hexylcyclohexyl)-tolan
4-Trifluormethoxy-4'-(trans-4-heptylcyclohexyl)-tolan
4-Trifluormethoxy-4'-(trans-4-octylcyclohexyl)-tolan
4-Trifluormethoxy-4'-ethyltolan
4-Trifluormethoxy-4'-propyltolan
4-Trifluormethoxy-4'-butyltolan
4-Trifluormethoxy-4'-pentyltolan
4-Trifluormethoxy-4'-hexyltolan
4-Trifluormethoxy-4'-heptyltolan
4-Trifluormethoxy-4'-octyltolan
4-Trifluormethoxy-4'-ethoxytolan
4-Trifluormethoxy-4'-propoxytolan
4-Trifluormethoxy-4'-butoxytolan
4-Trifluormethoxy-4'-pentyloxytolan
4-Trifluormethoxy-4'-hexyloxytolan
4-Trifluormethoxy-4'-heptyloxytolan
4-Trifluormethoxy-4'-octyloxytolan
4-Trifluormethoxy-4'-(4-ethylphenyl)tolan
4-Trifluormethoxy-4'-(4-propylphenyl)tolan
4-Trifluormethoxy-4'-(4-butylphenyl)tolan
4-Trifluormethoxy-4'-(4-pentylphenyl)tolan
4-Trifluormethoxy-4'-(4-hexylphenyl)tolan
4-Trifluormethoxy-4'-(4-heptylphenyl)tolan
4-Trifluormethoxy-4'-(4-octylphenyl)tolan
4-Trifluormethoxy-4'-(4-ethoxyphenyl)tolan
4-Trifluormethoxy-4'-(4-propoxyphenyl)tolan
4-Trifluormethoxy-4'-(4-butoxyphenyl)tolan
4-Trifluormethoxy-4'-(4-pentyloxyphenyl)tolan
4-Trifluormethoxy-4'-(4-hexyloxyphenyl)tolan
4-Trifluormethoxy-4'-(4-heptyloxyphenyl)tolan
4-Trifluormethoxy-4'-(4-octyloxyphenyl)tolan
1-(4-Trifluormethoxyphenyl)-2-[3-fluor-4-(4-ethylphenyl)-phenyl]-ethin
1-(4-Trifluormethoxyphenyl)-2-[3-fluor-4-(4-propylphenyl)-phenyl]-ethin
1-(4-Trifluormethoxyphenyl)-2-[3-fluor-4-(4-butylphenyl)-phenyl]-ethin
1-(4-Trifluormethoxyphenyl)-2-[3-fluor-4-(4-pentylphenyl)-phenyl]-ethin
1-(4-Trifluormethoxyphenyl)-2-[3-fluor-4-(4-ethoxyphenyl)-phenyl]-ethin
1-(4-Trifluormethoxyphenyl)-2-[3-fluor-4-(4-propoxyphenyl)-phenyl]-ethin
1-(4-Trifluormethoxyphenyl)-2-[3-fluor-4-(4-butyloxyphenyl)-phenyl]-ethin
1-(4-Trifluormethoxyphenyl)-2-[3-fluor-4-(4-pentyloxyphenyl)-phenyl]-ethin
4-Trifluormethoxy-4'-(trans-4-ethylcyclohexylethyl)-tolan
4-Trifluormethoxy-4'-(trans-4-propylcyclohexylethyl)-tolan
4-Trifluormethoxy-4'-(trans-4-butylcyclohexylethyl)-tolan
4-Trifluormethoxy-4'-(trans-4-pentylcyclohexylethyl)-tolan
4-Trifluormethoxy-4'-(trans-4-hexylcyclohexylethyl)-tolan
4-Trifluormethoxy-4'-(trans-4-heptylcyclohexylethyl)-tolan
4-Trifluormethoxy-4'-(trans-4-octylcyclohexylethyl)-tolan
1-(4-Trifluormethoxyphenyl)-2-[2,3-difluor-4-(4-ethyl-phenyl)-phenyl]-ethin
1-(4-Trifluormethoxyphenyl)-2-[2,3-difluor-4-(4-propyl-phenyl)-phenyl]-ethin
1-(4-Trifluormethoxyphenyl)-2-[2,3-difluor-4-(4-butyl-phenyl)-phenyl]-ethin
1-(4-Trifluormethoxyphenyl)-2-[2,3-difluor-4-(4-pentyl-phenyl)-phenyl]-ethin
1-(4-Trifluormethoxyphenyl)-2-[2,3-difluor-4-(4-hexyl-phenyl)-phenyl]-ethin
1-(4-Trifluormethoxyphenyl)-2-[2,3-difluor-4-(4-ethoxy-phenyl)-phenyl]-ethin
1-(4-Trifluormethoxyphenyl)-2-[2,3-difluor-4-(4-propoxy-phenyl)-phenyl]-ethin
1-(4-Trifluormethoxyphenyl)-2-[2,3-difluor-4-(4-butoxy-phenyl)-phenyl]-ethin
1-(4-Trifluormethoxyphenyl)-2-[2,3-difluor-4-(4-pentyloxy-phenyl)-phenyl]-ethin.

### Beispiel A

Eine flüssigkristalline Phase, bestehend aus
20 % p-trans-4-Pentylcyclohexyl-benzonitril,
18 % p-trans-4-Propylcyclohexyl-benzonitril,
20 % trans-1-p-Ethoxyphenyl-4-propylcyclohexan,
18 % trans-1-p-Butoxyphenyl-4-propylcyclohexan,
8 % 4-Methoxy-4'-(trans-4-propylcyclohexyl)-tolan,
8 % 4-Ethoxy-4'-(trans-4-propylcyclohexyl)-tolan und
8 % 4-Propyloxy-4-(trans-4-propylcyclohexyl)-tolan
hat einen Klärpunkt von + 85° und Δn = +0,154.

### Beispiel B

Eine flüssigkristalline Phase, bestehend aus
10 % 4-Ethyl-4'-cyanbiphenyl,
10 % 4-Propyl-4'-cyanbiphenyl,
10 % 4-Butyl-4'-cyanbiphenyl,
15 % 4-Pentyl-4'-cyanbiphenyl,
15 % 4-Hexyl-4'-cyanbiphenyl,
16 % trans-1-p-Methoxyphenyl-4-propylcyclohexan,
8 % 4-Methoxy-4'-(trans-4-propylcyclohexyl)-tolan,
8 % 4-Ethoxy-4'-(trans-4-propylcyclohexyl)-tolan, und
8 % 4-Propyloxy-4'-(trans-4-propylcyclohexyl)-tolan
hat einen Klärpunkt von +72° und Δn = +0,212.

### Beispiel C

Eine flüssigkristalline Phase, bestehend aus
10 % p-trans-4-Propylcyclohexyl-benzonitril,
18 % trans-1-p-Methoxyphenyl-4-propylcyclohexan,
15 % trans-1-p-Ethoxyphenyl-4-propylcyclohexan,
8 % 4-Fluor-4'-heptyloxy-tolan,
4 % 4-Methoxy-4'-(trans-4-propylcyclohexyl)-tolan,
3 % 4-Ethoxy-4'-(trans-4-propylcyclohexyl)-tolan,
5 % 4-Propyloxy-4'-(trans-4-propylcyclohexyl)-tolan,
12 % 4-Ethyl-4'-(trans-4-propylcyclohexyl)-biphenyl,
12 % 4-Ethyl-4'-(trans-4-pentylcyclohexyl)-biphenyl,
4 % 4,4'-Bis-(trans-4-propylcyclohexyl)-biphenyl,
5 % 4-(trans-4-Pentylcyclohexyl)-4'-(trans-4-propyl-cyclohexyl)-biphenyl und
4 % 4,4'-Bis-(trans-4-pentylcyclohexyl)-biphenyl
hat einen Klärpunkt von +110° und Δn = +0,160.

### Beispiel D

Eine flüssigkristalline Phase bestehend aus
13 % r-1-Cyan-cis-4-(trans-4-propylcyclohexyl)-1-propylcyclohexan,
21 % trans-1-p-Methoxyphenyl-4-propylcyclohexan,
24 % 1-(trans-4-Propylcyclohexyl)-2-(4'-ethyl-2'-fluorbiphenyl-4-yl)-ethan,
4 % 4,4'-Bis-(trans-4-propylcyclohexyl)-2-fluorbiphenyl,
4 % 4,4'-Bis-(trans-4-pentylcyclohexyl)-2-fluorbiphenyl,
4 % 4-(trans-4-Pentylcyclohexyl)-4'-(trans-4-propylcyclohexyl)-2-fluorbiphenyl,
12 % 4-Pentyl-4-propyltolan,
6 % 4-(trans-4-Propylcyclohexyl)4'-methoxytolan,
5 % 4-(trans-4-Propylcyclohexyl)4'-ethoxytolan und
7 % 4-(trans-4-Propylcyclohexyl)4'-propoxytolan
zeigt einen Klärpunkt von 101°, eine optische Anisotropie von 0,15 und eine Viskosität von 24 mPa.s bei 20°.

### Beispiel E

Eine flüssigkristalline Phase bestehend aus
13 % r-1-Cyan-cis-4-(trans-4-propylcyclohexyl)-1-propylcyclohexan,
19 % 1-(trans-4-Propylcyclohexyl)-2-(4'-ethyl-2'-fluor-biphenyl-4-yl)-ethan,
19 % 1-(trans-4-Propylcyclohexyl)-2-(4'-pentyl-2'-fluor-biphenyl-4-yl)-ethan,
16 % 1-(trans-4-Pentylcyclohexyl)-2-(4'-ethyl-2'-fluor-biphenyl-4-yl)-ethan,
5 % 4,4'-Bis-(trans-4-propylcyclohexyl)-2-fluor-biphenyl,
7 % 4-Butyl-4'-ethoxytolan,
7 % 4-Pentyl-4'-methoxytolan,
7 % 4-Pentyl-4'-ethoxytolan und
7 % 4-(trans-4-Propylcyclohexyl)-4'-methoxytolan
zeigt einen Klärpunkt von 99°, eine optische Anisotropie von 0,17 und eine Viskosität von 29 mPa.s bei 20°.

### Beispiel F

Eine flüssigkristalline Phase bestehend aus
12 % r-1-Cyan-cis-4-(trans-4-propylcyclohexyl)-1-propylcyclohexan,
21 % trans-1-p-Methoxyphenyl-4-propylcyclohexan,
21 % 1-(trans-4-Propylcyclohexyl)-2-(4'-ethyl-2'-fluor-biphenyl-4-yl)-ethan,
6 % 4,4'-Bis-(trans-4-propylcyclohexyl)-2-fluor-biphenyl,
6 % 4,4'-Bis-(trans-4-pentylcyclohexyl)-2-fluor-biphenyl,
6 % 4-(trans-4-Pentylcyclohexyl)-4'-(trans-4-propylcyclohexyl)-2-fluorbiphenyl,
7 % 4-Butyl-4'-ethoxytolan,
7 % 4-Pentyl-4'-methoxytolan,
7 % 4-Pentyl-4'-ethoxytolan und
7 % 4-(trans-4-Propylcyclohexyl)-4'-methoxytolan
zeigt einen Klärpunkt von 98°, eine optische Anisotropie von 0,16 und eine Viskosität von 24 mPa.s bei 20°.

### Beispiel G

Eine flüssigkristalline Phase bestehend aus
13 % r-1-Cyan-cis-4-(trans-4-propylcyclohexyl)-1-propylcyclohexan,
22 % trans-1-p-Methoxyphenyl-4-propylcyclohexan,
26 % 1-(trans-4-Propylcyclohexyl)-2-(4'-ethyl-2'-fluor-biphenyl-4-yl)-ethan,
6 % 4,4'-Bis-(trans-4-propylcyclohexyl)-2-fluor-biphenyl,
6 % 4,4'-Bis-(trans-4-pentylcyclohexyl)-2-fluor-biphenyl,
6 % 4-(trans-4-Pentylcyclohexyl)-4'-(trans-4-propylcyclohexyl)-2-fluorbiphenyl,
5 % 4-Butyl-4'-ethoxytolan,
5 % 4-Pentyl-4'-methoxytolan,
4 % 4-Pentyl-4'-ethoxytolan und
7 % 4-(trans-4'-Propylcyclohexyl)-4-methoxytolan
zeigt einen Klärpunkt von 99°, eine optische Anisotropie von 0,15 und eine Viskosität von 24 mPa.s bei 20°.

### Beispiel H

Eine flüssigkristalline Phase bestehend aus
3 % r-1-Cyan-cis-4-(trans-4-propylcyclohexyl)-1-propylcyclohexan,
20 % trans-1-p-Methoxyphenyl-4-propylcyclohexan,
8 % trans-1-p-Ethoxyphenyl-4-propylcyclohexan,
3 % 2-Fluor-4,4'-bis-(trans-4-propylcyclohexyl)-biphenyl,
5 % 2-Fluor-4-(trans-4-pentylcyclohexyl)-4'-(trans-4-propylcyclohexyl)-biphenyl,
16 % 4-Propyl-4'-butyl-tolan,
15 % 4-Propyl-4'-pentyl-tolan,
4 % 4-Methyl-4'-ethoxy-tolan,
5 % 4-Ethyl-4'-methoxy-tolan,
3 % 4-(trans-4-Propylcyclohexyl)-4'-ethoxytolan und
18 % 2,3-Difluor-4-ethoxy-4'-(trans-4-propylcyclohexyl)-tolan
hat einen Schmelzpunkt von < -40°, einen Klärpunkt von +80°, Δε = -0,9 und Δn = 0,2006.

### Beispiel I

Eine flüssigkristalline Phase bestehend aus
20 % 2,3-Difluor-4-ethoxy-4'-pentyl-tolan,
20 % trans-1-p-Methoxyphenyl-4-propylcyclohexan,
10 % trans-1-p-Ethoxyphenyl-4-propylcyclohexan,
5 % trans-1-p-Butoxyphenyl-4-propylcyclohexan,
4 % 2-Fluor-4,4'-bis(trans-4-propylcyclohexyl)-biphenyl,
4 % 2-Fluor-4-(trans-4-pentylcyclohexyl)-4'-(trans-4-propylcyclohexyl)-biphenyl,
5 % 4-Propyl-4'-butyl-tolan,
5 % 4-Propyl-4'-pentyl-tolan,
5 % 4-Methyl-4'-ethoxy-tolan,
4 % 4-Ethyl-4'-methoxy-tolan,
6 % 4-(trans-4-Propylcyclohexyl)-4'-methoxy-tolan,
5 % 4-(trans-4-Propylcyclohexyl)-4'-ethoxy-tolan und
7 % 4-(trans-4-Propylcyclohexyl)-4'-propoxy-tolan
hat einen Schmelzpunkt von < -40°, einen Klärpunkt von 85°, Δε = -1,1 und Δn = 0,2040.

## Patentansprüche

1. Flüssigkristalline Phase, dadurch gekennzeichnet, daß sie mindestens ein
Tolan der Formel I
R¹-(A¹-Z¹)ₘ-A³-C≡C-A^{⁴}-R² I
worin
R¹ und R² jeweils unabhängig voneinander Alkyl mit bis zu 15 C-Atomen, worin auch eine oder zwei nicht benachbarte CH₂-Gruppen durch -O-ersetzt sein können, einer der Reste R¹ und R² auch -OCF₃, CF₃, Fluor oder Chlor,
A¹ unsubstituiertes 1,4-Phenylen oder trans-1,4-Cyclohexylen,
Z¹ -CH₂CH₂- oder eine Einfachbindung,
m 0 oder 1
und
A³ und A⁴ jeweils unabhängig voneinander unsubstituiertes oder ein- oder mehrfach durch Fluor substituiertes 1,4-Phenylen
bedeutet, mit den Maßgaben, daß
entweder
a) die Tolane der Formel I eine 2,3-Difluor-1,4-phenylengruppe enthalten, oder
b) R¹ oder R² -CF₃ oder OCF₃ ist,
oder daß
c) im Falle m = 0 und A³ und A⁴ = unsubstituiertes 1,4-Phenylen R¹ Ethoxy, Propoxy, Butoxy, Pentoxy, Hexoxy oder Heptoxy und R² Fluor bedeutet oder einer der Reste R¹ und R² eine Alkylgruppe ist, worin mindestens zwei CH₂-Gruppen durch -O- ersetzt sind,
d) Verbindungen mit m = 0 und A³ und/oder A⁴ = monosubstituiertes 1,4-Phenylen ausgeschlossen sind,
e) im Falle A³ und A⁴ = unsubstituiertes 1,4-Phenylen, m = 1 und Z¹ = eine Einfachbindung, einer der Reste R¹ und R² eine Alkoxygruppe bedeutet und der andere dann Alkyl ist, und als wichtigste weitere Bestandteile Verbindungen der Formel V enthält,
R⁶-L-G-E-R⁷ V
worin L und E je ein carbo- oder heterocyclisches Ringsystem aus der aus 1,4-disubstituierten Cyclohexanringen, 4,4-disubstituierten Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,
| | | |
|---|---|---|
| G | -CH=CH- | -N(O)=N- |
| | -CH=CY- | -CH=N(O)- |
| | -C≡C | -CH₂-CH₂- |
| | -CO-O- | -CH₂-O- |
| | -CO-S- | -CH₂-S- |
| | -CH=N- | -COO-Phe-COO- |
oder eine C-C-Einfachbindung,
Y Chlor, oder -CN, und
R⁶ und R⁷ Alkyl, Alkoxy, Alkanoyloxy oder Alkoxycarbonyloxy mit bis zu 8 Kohlenstoffatomen, oder einer dieser Reste auch CN, NC, NO₂, CF₃, F, Cl oder Br bedeuten.

2. Flüssigkristalline Phase nach Anspruch 1, dadurch gekennzeichnet, daß sie Verbindungen ausgewählt aus den Teilformeln I5, I6, I18 und I22 enthält:
R¹-Cyc-Phe-C≡C-Phe-R² I5
R¹-Cyc-Phe-C≡C-Phex-R² I6
R¹-A¹-CH₂CH₂-Phe-C≡C-Phe-R² I18
R¹-A¹-CH₂CH₂-Phe-C≡C-Phex-R² I22
worin Cyc trans-1,4-Cyclohexylen, Phe unsubstituiertes 1,4-Phenylen und PheX ein- oder mehrfach durch Fluor substituiertes 1,4-Phenylen ist.

3. Tolane, gekennzeichnet durch die Teilformeln 2, 6 und 7: 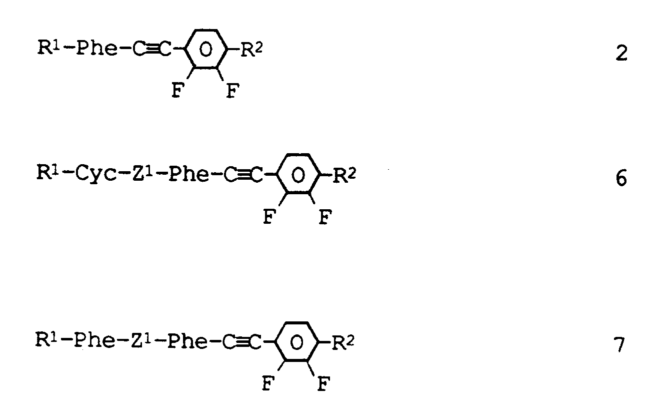 worin Cyc trans-1,4-Cyclohexylen und Phe unsubstituiertes 1,4-Phenylen bedeutet, R¹ und R² jeweils unabhängig voneinander Alkyl mit bis zu 15 C-Atomen, worin auch eine oder zwei nicht benachbarte CH₂-Gruppen durch -O- ersetzt sein können, einer der Reste R¹ und R² auch -OCF₃, CF₃, Fluor oder Chlor, und Z¹ -CH₂CH₂- oder eine Einfachbindung bedeutet.

4. Tolane, gekennzeichnet durch die Teilformel 21:
Alkyl-Cy-Phe-C≡C-Phe-Alkoxy 21
worin Cyc trans-1,4-Cyclohexylen und Phe unsubstituiertes 1,4-Phenylen bedeutet und Alkyl und Alkoxy jeweils bis zu 15 C-Atome aufweisen.

5. Tolane, gekennzeichnet durch die Teilformeln Igj und Igk:
R¹-Phe-C≡C-Phe-OCF₃ Igj
R¹-Cyc-Phe-C≡C-Phe-OCF₃ Igk
worin Cyc trans-1,4-Cyclohexylen, Phe unsubstituiertes 1,4-Phenylen und R¹ Alkyl mit bis zu 15 C-Atomen, worin auch eine oder zwei nicht benachbarte CH₂-Gruppen durch -O- ersetzt sein können, bedeutet.

6. 4-Methoxy-4'-(trans-4-propylcyclohexyl)-tolan
4-Ethoxy-4'-(trans-4-propylcyclohexyl)-tolan
4-Propyloxy-4'-(trans-4-propylcyclohexyl)-tolan

7. 4-Fluor-4'-ethoxy-tolan
4-Fluor-4'-propyloxy-tolan
4-Fluor-4'-butyloxy-tolan
4-Fluor-4'-pentyloxy-tolan
4-Fluor-4'-heptyloxy-tolan

8. 4-Methyl-4'-(2-methoxyethoxy)-tolan
4-Ethyl-4'-(2-methoxyethoxy)-tolan
4-Propyl-4'-(2-methoxyethoxy)-tolan
4-Butyl-4'-(2-methoxyethoxy)-tolan
4-Pentyl-4'-(2-methoxyethoxy)-tolan
4-Hexyl-4'-(2-methoxyethoxy)-tolan
4-Heptyl-4'-(2-methoxyethoxy)-tolan

9. 4-Fluor-4'-methoxymethoxy-tolan
4-Fluor-4'-(2-methoxyethoxy)-tolan

10. 2,3-Difluor-4-ethoxy-4'-(trans-4-methylcyclohexyl)-tolan
2,3-Difluor-4-ethoxy-4'-(trans-4-ethylcyclohexyl)-tolan
2,3-Difluor-4-ethoxy-4'-(trans-4-propylcyclohexyl)-tolan
2,3-Difluor-4-ethoxy-4'-(trans-4-butylcyclohexyl)-tolan
2,3-Difluor-4-ethoxy-4'-(trans-4-pentylcyclohexyl)-tolan
2,3-Difluor-4-ethoxy-4'-(trans-4-hexylcyclohexyl)-tolan
2,3-Difluor-4-ethoxy-4'-(trans-4-heptylcyclohexyl)-tolan

11. 2,3-Difluor-4-ethoxy-4'-methyltolan
2,3-Difluor-4-ethoxy-4'-ethyltolan
2,3-Difluor-4-ethoxy-4'-propyltolan
2,3-Difluor-4-ethoxy-4'-butyltolan
2,3-Difluor-4-ethoxy-4'-pentyltolan
2,3-Difluor-4-ethoxy-4'-hexyltolan
2,3-Difluor-4-ethoxy-4'-heptyltolan

12. 4-Trifluormethoxy-4'-(trans-4-ethylcyclohexyl)-tolan
4-Trifluormethoxy-4'-(trans-4-propylcyclohexyl)-tolan
4-Trifluormethoxy-4'-(trans-4-butylcyclohexyl)-tolan
4-Trifluormethoxy-4'-(trans-4-pentylcyclohexyl)-tolan
4-Trifluormethoxy-4'-(trans-4-hexylcyclohexyl)-tolan
4-Trifluormethoxy-4'-(trans-4-heptylcyclohexyl)-tolan
4-Trifluormethoxy-4'-(trans-4-octylcyclohexyl)-tolan
4-Trifluormethoxy-4'-ethyltolan
4-Trifluormethoxy-4'-propyltolan
4-Trifluormethoxy-4'-butyltolan
4-Trifluormethoxy-4'-pentyltolan
4-Trifluormethoxy-4'-hexyltolan
4-Trifluormethoxy-4'-heptyltolan
4-Trifluormethoxy-4'-octyltolan
4-Trifluormethoxy-4'-ethoxytolan
4-Trifluormethoxy-4'-propoxytolan
4-Trifluormethoxy-4'-butoxytolan
4-Trifluormethoxy-4'-pentyloxytolan
4-Trifluormethoxy-4'-hexyloxytolan
4-Trifluormethoxy-4'-heptyloxytolan
4-Trifluormethoxy-4'-octyloxytolan

13. Flüssigkristalline Phase mit mindestens zwei flüssigkristallinen Komponenten, dadurch gekennzeichnet, daß sie mindestens eine Verbindung nach einem der Ansprüche 3 bis 12 enthält.

14. Verwendung der Verbindungen nach einem der Ansprüche 3 bis 12 als Komponenten flüssigkristalliner Phasen.

15. Verwendung der Verbindungen nach einem der Ansprüche 3 bis 12 als Komponenten flüssigkristalliner Phasen für elektrooptische Anzeigeelemente basierend auf dem ECB-Effekt.

16. Flüssigkristalline Phase für elektrooptische Anzeigeelemente basierend auf dem ECB-Effekt mit mindestens zwei flüssigkristallinen Komponenten, dadurch gekennzeichnet, daß sie mindestens eine Verbindung nach einem der Ansprüche 3 bis 12 enthält.

17. Elektrooptisches Anzeigeelement, dadurch gekennzeichnet, daß es als flüssigkristallines Dielektrikum eine flüssigkristalline Phase nach Anspruch 1, 2 oder 13 enthält.

18. Elektrooptisches Anzeigeelement basierend auf dem ECB-Effekt, dadurch gekennzeichnet, daß es als Dielektrikum eine Phase nach Anspruch 16 enthält.

19. Tolane der Formel I
R¹-(A¹-Z¹)ₘ-A³-C≡C-A⁴-R² I
worin
R¹ Alkyl mit bis zu 15 C-Atomen,
R² -OCF₃
A¹ trans-1,4-Cyclohexylen,
Z¹ eine Einfachbindung,
m 1
und
A³ und A⁴ jeweils unabhängig voneinander unsubstituiertes oder ein- oder mehrfach durch Fluor substituiertes 1,4-Phenylen
bedeutet.

20. Tolane der Formel I nach Anspruch 19, worin R¹ eine geradkettige Alkylgruppe mit 1-7 C-Atomen bedeutet.

## Claims

1. Liquid-crystalline phase, characterised in that it contains at least one tolan of the formula I
R¹-(A¹-Z¹)ₘ-A³-C≡C-A⁴-R² I
in which
R¹ and R² are each, independently of one another, alkyl having up to 15 carbon atoms in which, in addition, one or two non-adjacent CH₂ groups may be replaced by -O-, and one of the radicals R¹ and R² is alternatively -OCF₃, CF₃, fluorine or chlorine,
A¹ is unsubstituted 1,4-phenylene or trans-1,4-cyclohexylene,
Z¹ is -CH₂CH₂- or a single bond,
m is 0 or 1,
and
A³ and A⁴ are each, independently of one another, 1,4-phenylene which is unsubstituted or monosubstituted or polysubstituted by fluorine,
with the provisos that either
a) the tolans of the formula I contain a 2,3-difluoro-1,4-phenylene group, or
b) R¹ or R² is -CF₃ or OCF₃,
or that
c) in the case where m = 0 and A³ and A⁴ = unsubstituted 1,4-phenylene, R¹ is ethoxy, propoxy, butoxy, pentoxy, hexoxy or heptoxy, and R² is fluorine, or one of the radicals R¹ and R² is an alkyl group in which at least two CH₂ groups have been replaced by -O-,
d) compounds where m = 0 and A³ and/or A⁴ = monosubstituted 1,4-phenylene are excluded,
e) in the case where A³ and A⁴ = unsubstituted 1,4-phenylene, m = 1 and Z¹ = a single bond, one of the radicals R¹ and R² is an alkoxy group and the other is then alkyl,
and contains, as the most important further constituents, compounds of the formula V
R⁶-L-G-E-R⁷ V
in which L and E are each a carbocyclic or heterocyclic ring system from the group formed by 1,4-disubstituted cyclohexane rings, 4,4-disubstituted phenylcyclohexane and cyclohexylcyclohexane systems, 2,5-disubstituted pyrimidine and 1,3-dioxane rings, 2,6-disubstituted naphthaline, di- and tetrahydronaphthaline, quinazoline and tetrahydroquinazoline,
| | | |
|---|---|---|
| G is | -CH=CH- | -N(O)=N- |
| | -CH=CY- | -CH=N(O)- |
| | -C=C | -CH₂-CH₂- |
| | -CO-O- | -CH₂-O- |
| | -CO-S- | -CH₂-S- |
| | -CH=N- | -COO-Phe-COO- |
or a C-C single bond,
Y is chlorine or -CN, and
R⁶ and R⁷ are alkyl, alkoxy, alkanoyloxy or alkoxycarbonyloxy having up to 8 carbon atoms, or one of these radicals is alternatively CN, NC, NO₂, CF₃, F, Cl or Br.

2. Liquid-crystalline phase according to Claim 1, characterised in that it contains compounds selected from the sub-formulae I5, I6, I18 and I22:
R¹-Cyc-Phe-C≡C-Phe-R² I5
R¹-Cyc-Phe-C≡C-PheX-R² I6
R¹-A¹-CH₂CH₂-Phe-C≡C-Phe-R² I18
R¹-A¹-CH₂CH₂-Phe-C≡C-PheX-R² I22
in which Cyc is trans-1,4-cyclohexylene, Phe is unsubstituted 1,4-phenylene and PheX is 1,4-phenylene which is monosubstituted or polysubstituted by fluorine.

3. Tolans, characterised by the sub-formulae 2, 6 and 7: 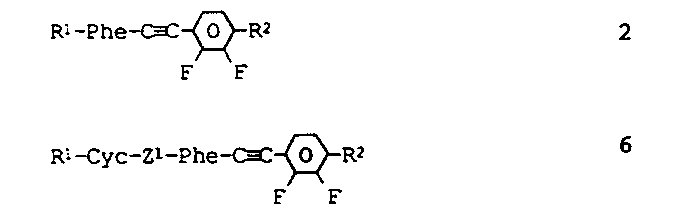 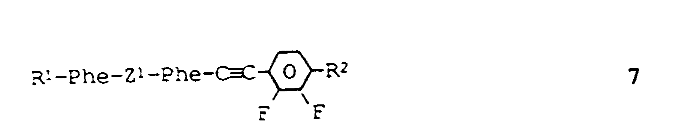 in which Cyc is trans-1,4-cyclohexylene and Phe is unsubstituted 1,4-phenylene, R¹ and R² are each, independently of one another, alkyl having up to 15 carbon atoms in which, in addition, one or two non-adjacent CH₂ groups may be replaced by -O-, and one of the radicals R¹ and R² is alternatively -OCF₃, CF₃, fluorine or chlorine, and Z¹ is -CH₂CH₂- or a single bond.

4. Tolans, characterised by the sub-formula 21:
Alkyl-Cy-Phe-C≡C-Phe-alkoxy 21
in which Cyc is trans-1,4-cyclohexylene, and Phe is unsubstituted 1,4-phenylene and alkyl and alkoxy each have up to 15 carbon atoms.

5. Tolans, characterised by the sub-formulae Igj and Igk:
R¹-Phe-C≡C-Phe-OCF₃ Igj
R¹-Cyc-Phe-C≡C-Phe-OCF₃ Igk
in which Cyc is 1,4-cyclohexylene, Phe is unsubstituted 1,4-phenylene, and R¹ is alkyl having up to 15 carbon atoms in which, in addition, one or two non-adjacent CH₂ groups may be replaced by -O-.

6. 4-Methoxy-4'-(trans-4-propylcyclohexyl)tolan
4-Ethoxy-4'-(trans-4-propylcyclohexyl)tolan
4-Propyloxy-4'-(trans-4-propylcyclohexyl)tolan

7. 4-Fluoro-4'-ethoxytolan
4-Fluoro-4'-propyloxytolan
4-Fluoro-4'-butyloxytolan
4-Fluoro-4'-pentyloxytolan
4-Fluoro-4'-heptyloxytolan

8. 4-Methyl-4'-(2-methoxyethoxy)tolan
4-Ethyl-4'-(2-methoxyethoxy)tolan
4-Propyl-4'-(2-methoxyethoxy)tolan
4-Butyl-4'-(2-methoxyethoxy)tolan
4-Pentyl-4'-(2-methoxyethoxy)tolan
4-Hexyl-4'-(2-methoxyethoxy)tolan
4-Heptyl-4'-(2-methoxyethoxy)tolan

9. 4-Fluoro-4'-methoxymethoxytolan
4-Fluoro-4'-(2-methoxyethoxy)tolan

10. 2,3-Difluoro-4-ethoxy-4'-(trans-4-methlcyclohexyl)tolan
2,3-Difluoro-4-ethoxy-4'-(trans-4-ethylcyclohexyl)tolan
2,3-Difluoro-4-ethoxy-4'-(trans-4-propylcyclohexyl)tolan
2,3-Difluoro-4-ethoxy-4'-(trans-4-butylcyclohexyl)tolan
2,3-Difluoro-4-ethoxy-4'-(trans-4-pentylcyclohexyl)tolan
2,3-Difluoro-4-ethoxy-4'-(trans-4-hexylcyclohexyl)tolan
2,3-Difluoro-4-ethoxy-4'-(trans-4-heptylcyclohexyl)tolan

11. 2,3-Difluoro-4-ethoxy-4'-methyltolan
2,3-Difluoro-4-ethoxy-4'-ethyltolan
2,3-Difluoro-4-ethoxy-4'-propyltolan
2,3-Difluoro-4-ethoxy-4'-butyltolan
2,3-Difluoro-4-ethoxy-4'-pentyltolan
2,3-Difluoro-4-ethoxy-4'-hexyltolan
2,3-Difluoro-4-ethoxy-4'-heptyltolan

12. 4-Trifluoromethoxy-4'-(trans-4-ethylcyclohexyl)tolan
4-Trifluoromethoxy-4'-(trans-4-propylcyclohexyl)tolan
4-Trifluoromethoxy-4'-(trans-4-butylcyclohexyl)tolan
4-Trifluoromethoxy-4'-(trans-4-pentylcyclohexyl)tolan
4-Trifluoromethoxy-4'-(trans-4-hexylcyclohexyl)tolan
4-Trifluoromethoxy-4'-(trans-4-heptylcyclohexyl)tolan
4-Trifluoromethoxy-4'-(trans-4-octylcyclohexyl)tolan
4-Trifluoromethoxy-4'-ethyltolan
4-Trifluoromethoxy-4'-propyltolan
4-Trifluoromethoxy-4'-butyltolan
4-Trifluoromethoxy-4'-pentyltolan
4-Trifluoromethoxy-4'-hexyltolan
4-Trifluoromethoxy-4'-heptyltolan
4-Trifluoromethoxy-4'-octyltolan
4-Trifluoromethoxy-4'-ethoxytolan
4-Trifluoromethoxy-4'-propoxytolan
4-Trifluoromethoxy-4'-butoxytolan
4-Trifluoromethoxy-4'-pentyloxytolan
4-Trifluoromethoxy-4'-hexyloxytolan
4-Trifluoromethoxy-4'-heptyloxytolan
4-Trifluoromethoxy-4'-octyloxytolan

13. Liquid-crystalline phase having at least two liquid-crystalline components, characterised in that it contains at least one compound according to one of Claims 3 to 12.

14. Use of the compounds according to one of Claims 3 to 12 as components of liquid-crystalline phases.

15. Use of compounds according to one of Claims 3 to 12 as components of liquid-crystalline phases for electro-optical display elements based on the ECB effect.

16. Liquid-crystalline phase for electro-optical display elements based on the ECB effect, having at least two liquid-crystalline components, characterised in that it contains at least one compound according to one of Claims 3 to 12.

17. Electro-optical display element, characterised in that it contains, as liquid-crystalline dielectric, a liquid-crystalline phase according to Claim 1, 2 or 13.

18. Electro-optical display element based on the ECB effect, characterised in that it contains, as dielectric, a phase according to Claim 16.

19. Tolans of the formula I
R¹-(A¹-Z¹)m-A³-C≡C-A⁴-R² I
in which
R¹ is alkyl having up to 15 carbon atoms,
R² is -OCF₃,
A¹ is trans-1,4-cyclohexylene,
Z¹ is a single bond,
m is 1
and
A³ and A⁴ are each, independently of one another, 1,4-phenylene which is unsubstituted or monosubstituted or polysubstituted by fluorine.

20. Tolans of the formula I according to Claim 19, in which R¹ is a straight-chain alkyl group having 1-7 carbon atoms.

## Revendications

1. Phase cristalline liquide, caractérisée en ce qu'elle contient au moins un
tolane de formule I
R¹-(A¹-Z¹)ₘ-A³-C≡C-A⁴-R² I
dans laquelle
R¹ et R² représentent chacun indépendamment l'un de l'autre un alcoyle en C₁ à C₁₅ , où également un ou deux groupes CH₂ non voisins peuvent être remplacés par -O-, l'un des radicaux R¹ et R² représente également -OCF₃ , CF₃ , fluor ou chlore,
A¹ représente un 1,4-phénylène ou trans-1,4-cyclohexylène non substitué,
Z¹ représente -CH₂CH₂ ou une liaison simple,
m vaut 0 ou 1
et
A³ et A⁴ représentent chacun indépendamment l'un de l'autre un 1,4-phénylène non substitué ou mono- ou polysubstitué par un fluor,
avec ces précisions que
ou bien
a) les tolanes de formule I contiennent un groupe 2,3-difluoro-1,4-phénylène, ou
b) R¹ ou R² représente -CF₃ ou OCF₃,
ou bien
c) dans le cas où m = 0 et A³ et A⁴ = 1,4-phénylène non substitué, R¹ représente un éthoxy, propoxy, butoxy, pentoxy, hexoxy ou heptoxy et R² représente un fluor ou l'un des radicaux R¹ et R² est un groupe alcoyle, où au moins deux groupes CH₂ sont remplacés par -O-,
d) les composés où m = 0 et A³ et/ou A⁴ 1,4-phénylène monosubstitué sont exclus,
e) dans le cas où A³ et A⁴ = 1,4-phènylène non substitué, m = 1 et Z¹ = une liaison simple, l'un des radicaux R¹ et R² représente un groupe alcoxy et l'autre est un alcoyle,
et comme autres composants très importants des composés de formule V
R⁶-L-G-E-R⁷ V
où L et E représentent chacun un système cyclique carbo- ou hétérocyclique parmi les suivants: noyaux cyclohexane 1,4-disubstitués, systèmes phényl-cyclohexane et cyclohexyl-cyclohexane 4,4-disubstitués, noyaux pyrimidine et 1,3-dioxanne 2,5-disubstitués, naphtalène 2,6-disubstitué, di- et tétrahydronaphtalène, quinazoline et tétrahydroquinazoline,
| | | |
|---|---|---|
| G | -CH=CH- | -N(O)=N- |
| | -CH=CY- | -CH=N(O)- |
| | -C≡C | -CH₂-CH₂- |
| | -CO-O- | -CH₂-O- |
| | -CO-S- | -CH₂-S- |
| | -CH=N- | -COO-Phe-COO- |
ou une liaison simple C-C,
Y un chlore au -CN, et
R⁶ et R⁷ représentent un alcoyle, alcoxy, alcanoyloxy ou alcoxycarbonyloxy avec jusqu'à 8 atomes de carbone, ou un de ces radicaux représente également CN, NC, NO₂ CF₃ , F , Cl ou Br.

2. Phase cristalline liquide selon la revendication 1, caractérisée en ce qu'elle contient dos composés de formules partielles 15, 16, 118 et 122:
R¹-Cyc-Phe-C≡C-Phe-R² I5
R¹-Cyc-Phe-C≡C-PheX-R² I6
R¹-A¹-CH₂CH₂-Phe-C≡C-Phe-R² I18
R¹-A¹-CH₂CH₂-Phe-C≡C-PheX-R² I22
où Cyc représente un trans-1,4-cyclohexylène, Phe un 1,4-phénylène non substitué et PheX est un 1,4-phénylène mono- ou polysubstitué par un fluor.

3. Tolanes caractérisés par les formules partielles 2, 6 et 7; 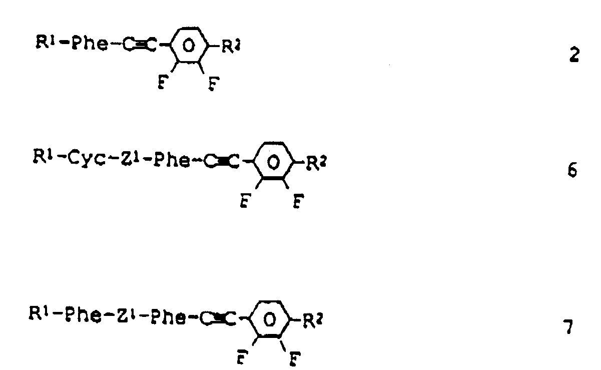 où Cyc représente un trans-1,4-cyclohexylène et Phe un 1,4-phénylène non substitué, R¹ et R² représentent à chaque fois indépendamment l'un de l'autre un alcoyle ayant jusqu'à 15 atomes de carbone, où également un ou deux groupes CH₂ non voisins peuvent être remplacés par -O-, l'un des radicaux R¹ et R² représente par -O-, l'un des radicaux R¹ et R² représente également -OCF₃ CF₃ , un fluor ou un chlore, et Z¹ représente -CH₂CH₂- ou une liaison simple.

4. Tolanes caractérisés par la formule partielle 21:
alcoyl-Cyc-Phe-C≡C-Phe-alcoxy 21
où Cyc représente un trans-1,4-cyclohexylène et Phe un 1,4-phénylène non substitué et alcoyle et alcoxy présentent à chaque fois jusqu'à 15 atomes de carbone.

5. Tolanes caractérisés par les formules partielles Igj et Igk:
R¹-Phe-C≡C-Phe-OCF₃ Igj
R¹-Cyc-Phe-C≡C-Phe-OCF₃ Igk
où Cyc représente un trans-1,4-cyclohexylène, Phe un 1,4-phénylène non substitué et R¹ un alcoyle ayant jusqu'à 15 atomes de carbone, où également un ou deux groupes CH₂ non voisins peuvent être remplacés par -O-.

6. 4-Méthoxy-4'-(trans-4-propylcyclohexyl)-tolane
4-Ethoxy-4'-(trans-4-propylcyclohexyl)-tolane
4-Propyloxy-4'-(trans-4-propylcyclohexyl)-tolane

7. 4-Fluoro-4'-éthoxy-tolane
4-Fluoro-4'-propyloxy-tolane
4-Fluoro-4'-butyloxy-tolane
4-Fluoro-4'-pentyloxy-tolane
4-Fluoro-4'-heptyloxy-tolane

8. 4-Méthyl-4'-(2-méthoxyéthoxy)-tolane
4-Ethyl-4'-(2-méthoxyéthoxy)-tolane
4-Propyl-4'-(2-méthoxyéthoxy)-tolane
4-Butyl-4'-(2-méthoxyéthoxy)-tolane
4-Pentyl-4'-(2-méthoxyéthoxy)-tolane
4-Hexyl-4'-(2-méthoxyéthoxy)-tolane
4-Heptyl-4'-(2-méthoxyéthoxy)-tolane

9. 4-Fluoro-4'-méthoxyméthoxy-tolane
4-Fluoro-4'-(2-méthoxyéthoxy)-tolane

10. 2,3-Difluoro-4-éthoxy-4'-(trans-4-méthylcyclohexyl)-tolane
2,3-Difluoro-4-éthoxy-4'-(trans-4-éthylcyclohexyl)-tolane
2,3-Difluoro-4-éthoxy-4'-(trans-4-propylcyclohexyl)-tolane
2,3-Difluoro-4-éthoxy-4'-(trans-4-butylcyclohexyl)-tolane
2,3-Difluoro-4-éthoxy-4'-(trans-4-pentylcyclohexyl)-tolane
2,3-Difluoro-4-éthoxy-4'-(trans-4-hexylcyclohexyl)-tolane.
2,3-Difluoro-4-éthoxy-4'-(trans-4-heptylcyclohexyl)-tolane

11. 2,3-Difluoro-4-éthoxy-4'-méthyltolane
2,3-Difluoro-4-éthoxy-4'-éthyltolane
2,3-Difluoro-4-éthoxy-4'-propyltolane
2,3-Difluoro-4-éthoxy-4'-butyltolane
2,3-Difluoro-4-éthoxy-4'-pentyltolane
2,3-Difluoro-4-éthoxy-4'-hexyltolane
2,3-Difluoro-4-éthoxy-4'-heptyltolane

12. 4-Trifluorométhoxy-4'-(trans-4-éthylcyclohexyl)-tolane
4-Trifluorométhoxy-4-(trans-4-propylcyclohexyl)-tolane
4-Trifluorométhoxy-4'-(trans-4-butylcyclohexyl)-tolane
4-Trifluorométhoxy-4'-(trans-4-pentylcyclohexyl)-tolane.
4-Trifluorométhoxy-4'-(trans-4-hexylcyclohexyl)-tolane
4-Trifluorométhoxy-4'-(trans-4-heptylcyclohexyl)-tolane
4-Trifluorométhoxy-4'-(trans-4-octylcyclohexyl)-tolane
4-Trifluorométhoxy-4'-éthyltolane
4-Trifluorométhoxy-4'-propyltolane
4-Trifluorométhoxy-4'-butyltolane
4-Trifluorométhoxy-4'-pentyltolane
4-Trifluorométhoxy-4'-hexyltolane
4-Trifluorométhoxy-4'-heptyltolane
4-Trifluorométhoxy-4'-octyltolane
4-Trifluorométhoxy-4'-éthoxytolane
4-Trifluorométhoxy-4'-propoxytolane
4-Trifluorométhoxy-4'-butoxytolane
4-Trifluorométhoxy-4'-pentyloxytolane
4-Trifluorométhoxy-4'-hexyloxytolane
4-Trifluorométhoxy-4'-heptyloxytolane
4-Trifluorométhoxy-4'-octyloxytolane

13. Phase cristalline liquide avec au moins deux composants cristallins liquides, caractérisée en ce qu'elle contient au moins un composé selon l'une des revendications 3 à 12.

14. Application des composés selon l'une des revendications 3 à 12 comme composants de phases cristallines liquides.

15. Application des composés selon l'une des revendications 3 à 12 comme composants de phases cristallines liquides pour éléments d'affichage électro-optiques à base d'effet ECB.

16. Phase cristalline liquide pour éléments d'affichage électro-optiques à base d'effet ECB avec au moins deux composants cristalline liquides, caractérisée en ce qu'elle contient au moins un composé selon l'une des revendications 3 à 12.

17. Elément d'affichage électro-optique caractérisé en ce qu'il contient comme diélectrique cristallin liquide une phase cristalline liquide selon la revendication 1, 2 ou 13.

18. Elément d'affichage électro-optique à base d'effet ECB, caractérisé en ce qu'il contient comme diélectrique une phase selon la revendication 16.

19. Tolanes de formule I
R¹-(A¹-Z¹)ₘ-A³-C≡C-A⁴-R² I
dans laquelle
R¹ représente un alcoyle ayant jusqu'à 15 atomes de carbone,
R² représente -OCF₃
A¹ un trans-1,4-cyclohexylène,
Z¹ une liaison simple,
m vaut 1
et
A³ et A⁴ représentent chacun indépendamment l'un de l'autre un 1,4-phénylène non substitué ou mono- ou polysubstitué par un fluor.

20. Tolanes de formule I selon la revendication 19, où R¹ représente un groupe alcoyle en C₁ à C₇.
